# EUROPEAN PATENT SPECIFICATION

(11) **EP 3 552 022 B1**
(45) Date of publication and mention of the grant of the patent: **09.06.2021**
(21) Application number: 17812095.2
(22) Date of filing: 24.11.2017
(51) Int. Cl.: G01N 33/68

(54) **METABOLIC DISORDERS**
STOFFWECHSELERKRANKUNGEN
TROUBLES MÉTABOLIQUES

(30) Priority: 25.11.2016 SE 1651551
(43) Date of publication of application: 16.10.2019
(73) Proprietor: Implexion AB, 411 26 Gothenburg (SE)
(72) Inventor: BÄCKHED, Fredrik, 429 35 Kullvik (SE); KOH, Ara, 413 26 Göteborg (SE)
(74) Representative: Barker Brettell Sweden AB
(86) International application number: PCT/SE2017/051169
(87) International publication number: WO 2018/097793

(56) References cited:
- WO-A1-2006/008342
- WO-A1-2014/118634
- US-A1- 2016 266 148
- YU DANXIA ET AL: "Plasma metabolomic profiles in association with type 2 diabetes risk and prevalence in Chinese adults", METABOLOMICS, SPRINGER NEW YORK LLC, US, vol. 12, no. 1, 7 November 2015 (2015-11-07), pages 1-11, XP035936324, ISSN: 1573-3882, DOI: 10.1007/S11306-015-0890-8 [retrieved on 2015-11-07]
- MUHAMMAD TANWEER KHAN ET AL: "Microbial Modulation of Insulin Sensitivity", CELL METABOLISM, vol. 20, no. 5, 1 November 2014 (2014-11-01), pages 753-760, XP055440939, United States ISSN: 1550-4131, DOI: 10.1016/j.cmet.2014.07.006
- F. KARLSSON ET AL: "Assessing the Human Gut Microbiota in Metabolic Diseases", DIABETES, vol. 62, no. 10, 24 September 2013 (2013-09-24), pages 3341-3349, XP055440944, US ISSN: 0012-1797, DOI: 10.2337/db13-0844
- ANNICK V. HARTSTRA ET AL: "Insights Into the Role of the Microbiome in Obesity and Type 2 Diabetes", DIABETES CARE, vol. 38, no. 1, 23 December 2014 (2014-12-23), pages 159-165, XP055440956, US ISSN: 0149-5992, DOI: 10.2337/dc14-0769

## Description

### TECHNICAL FIELD

The present embodiments generally relate to metabolic disorders, and in particular to type 2 diabetes and impaired glucose tolerance and to diagnosing and treating such metabolic disorders.

### BACKGROUND

Metabolic disorders, such as obesity, diabetes and cardiovascular disease, are associated with altered gut microbiota structure and function, and recent data suggest that the microbiota contributes to variations in metabolic responses to a given diet. Studies assessing the effect of diet interventions on the microbiota have mostly focused on dietary fibers, which are precursors for bacterial fermentation, resulting in metabolites, such as short-chain fatty acids, which are associated with improved metabolism. Furthermore, recent studies have identified trimethylamine (TMA) N-oxide (TMAO) as a microbiota-dependent metabolite that contributes to atherosclerosis in mouse models. TMAO is formed from the dietary precursors choline, phosphatidylcholine and carnitine in a two-step process involving microbial TMA lyase and host hepatic flavin monooxygenases. Attempts to inhibit microbial TMA lyase as a potential therapeutic approach against atherosclerosis are currently underway.

Although the gut microbiota has profound effects on amino acid metabolism and can regulate the levels of amino acid-derived signaling molecules, such as neurotransmitters and serotonin, the contribution of microbially produced amino acid metabolites to host metabolism is controversial. The microbiota has been reported to contribute to increased circulating levels of branched-chain amino acids (BCAA) in obese or insulin-resistant individuals, but several studies have demonstrated improved metabolic health following dietary BCAA supplementation. To date, there is no direct evidence to indicate that microbiota-dependent amino acid-derived metabolites contribute to metabolic disorders.

Yu et al., Plasma metabolomics profiles in association with type 2 diabetes risk and prevalence in Chinese adults, Metabolomics, 12(1): 1-11 (2015) identified 36 known metabolites and 10 unknown metabolites associated with prevalent and/or incident type 2 diabetes (T2D). The known metabolites were involved in metabolic pathways of glycolysis/gluconeogenesis, branched-chain amino acids, other amino acids, fatty acids, glycerophospholipids, androgen, and bradykinin. The findings suggest that hexoses, branched-chain amino acids, and yet to be validated novel plasma metabolites may improve risk prediction and mechanistic understanding of T2D in Chinese populations.

### SUMMARY

It is a general objective to identify subjects suffering from, or having a risk of suffering from, type 2 diabetes (T2D) or impaired glucose tolerance (IGT).

It is another general objective to identify and use agents effective in preventing, inhibiting or treating T2D or IGT.

These and other objectives are met by embodiments disclosed herein.

The invention is defined in the independent claims. Further embodiments of the invention are defined in the dependent claims.

An aspect of the embodiments relates to a method of determining whether a subject is suffering from, or having a risk of suffering from, T2D or IGT. The method comprises determining an amount of imidazole propionate and urocanate in a body sample from the subject. The method also comprises determining whether the subject is suffering from, or having a risk of suffering from, T2D or IGT based on the determined amount of imidazole propionate and urocanate.

An aspect of the embodiments relates to a method of determining whether a subject is suffering from, or having a risk of suffering from, T2D or IGT. The method comprises determining an amount of imidazole propionate producing bacteria in a body sample from the subject. The method also comprises determining whether the subject is suffering from, or having a risk of suffering from, type 2 diabetes or impaired glucose tolerance based on the determined amount of imidazole propionate producing bacteria.

A further aspect of the embodiments relates to a method of identifying an agent effective in preventing, inhibiting or treating T2D or IGT in a subject. The method comprises adding an agent to an *in vitro* simulated human intestinal model challenged with histidine and/or urocanate. The method also comprises measuring an amount of imidazole propionate produced by the *in vitro* simulated human intestinal model. The method further comprises identifying the agent as effective in preventing, inhibiting or treating T2D or IGT based on the amount of imidazole propionate.

The embodiments are based on the experimental results showing that microbially produced imidazole propionate impairs insulin signaling by activating alternative p38 MAPK in humans suffering from T2D or IGT.

### BRIEF DESCRIPTION OF THE DRAWINGS

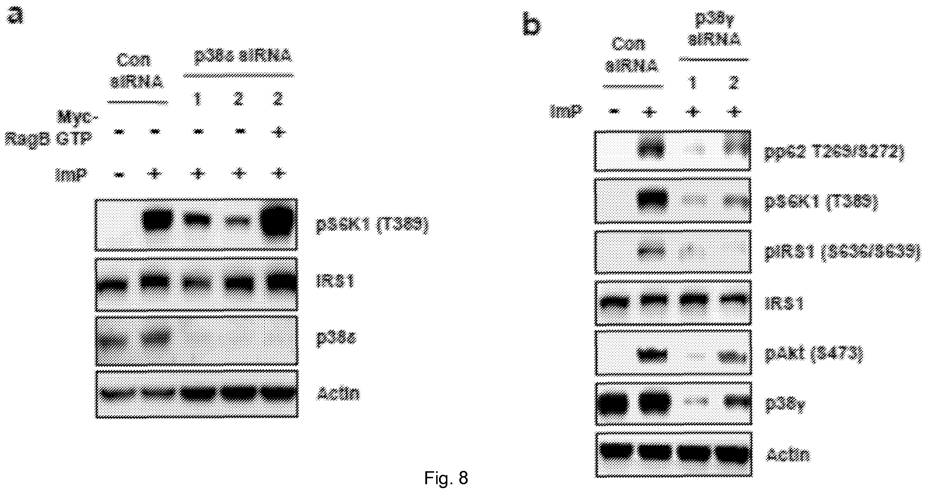

The embodiments, together with further objects and advantages thereof, may best be understood by making reference to the following description taken together with the accompanying drawings, in which:

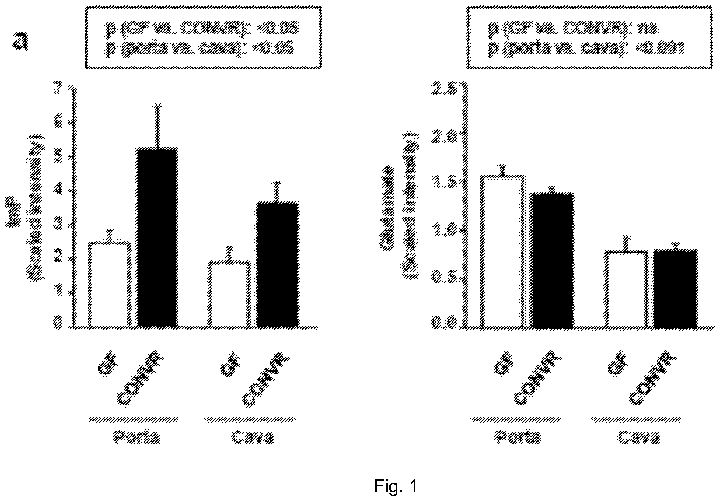

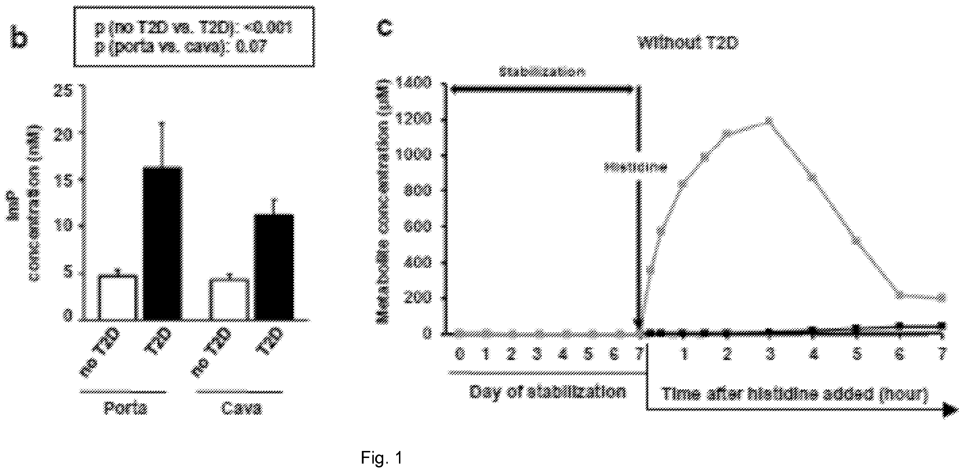

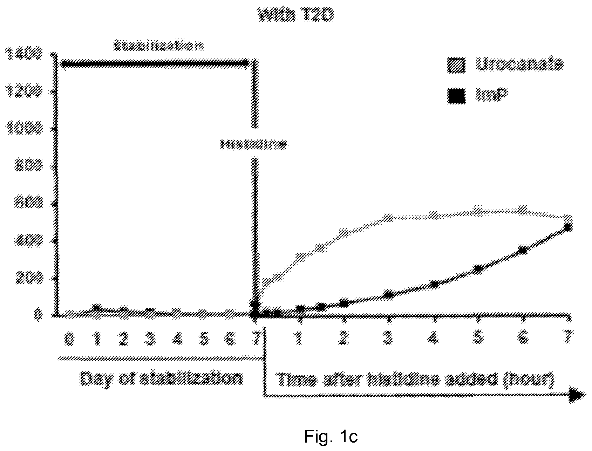

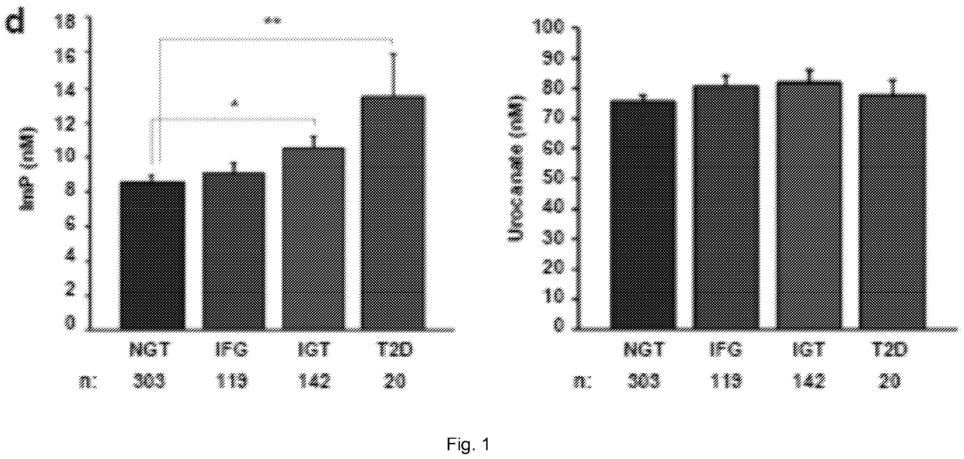

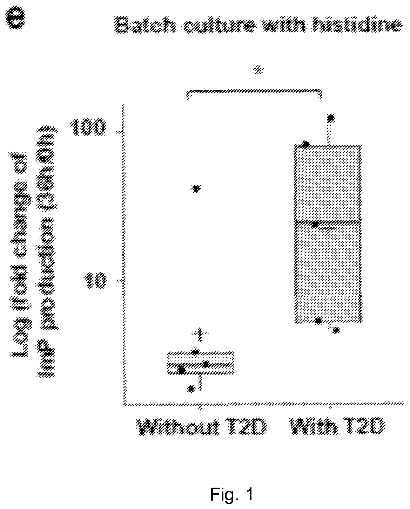

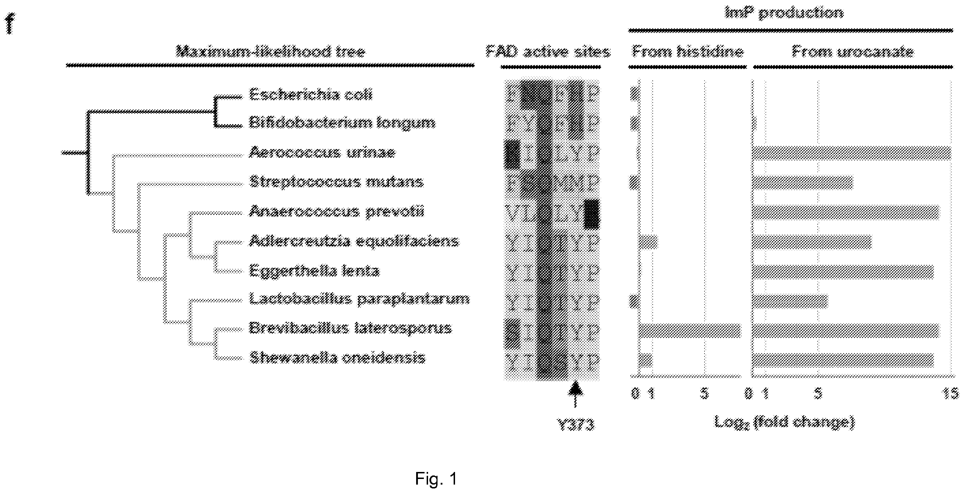

Fig. 1. Imidazole propionate is increased in individuals with type 2 diabetes and is microbially regulated. Fig. 1a, Relative levels of imidazole propionate (ImP) and glutamate in portal and vena cava blood of GF and CONVR mice (n = 6 per group). Fig. 1b, Portal and peripheral levels of ImP in obese BMI-matched subjects without type 2 diabetes (no T2D, *n* = 10) or with T2D (*n* = 5). Fig. 1c, Production of urocanate and ImP from 10 mM histidine in an *in vitro* gut simulator inoculated with feces from a human without T2D (left panel) or from a human with T2D (right panel). Fig. 1d, Peripheral levels of ImP or urocanate in humans with normal glucose tolerance (NGT), impaired fasting glucose (IFG), impaired glucose tolerance (IGT) and treatment-naive T2D. Fig. 1e, End-point measurements of ImP production from 10 mM histidine in batch culture experiments using feces from subjects without T2D (n = 5) and from subjects with T2D (n = 5). Data are shown as log transformed fold-change of ImP production at 36 h compared with at 0 h. Fig. 1f, Prediction of imidazole propionate-producing bacteria and experimental validation using histidine or urocanate as substrate. ImP production shown as log transformed fold change compared with blank samples. Data in Figs. 1a, 1b and 1d are mean ± s.e.m. Two-way ANOVA with repeated measurements (Figs. 1a, 1b). **P* < 0.05, ***P* < 0.01; Wilcoxon Rank-Sum test (Fig. 1d); Wilcoxon one-tailed test (Fig. 1e).

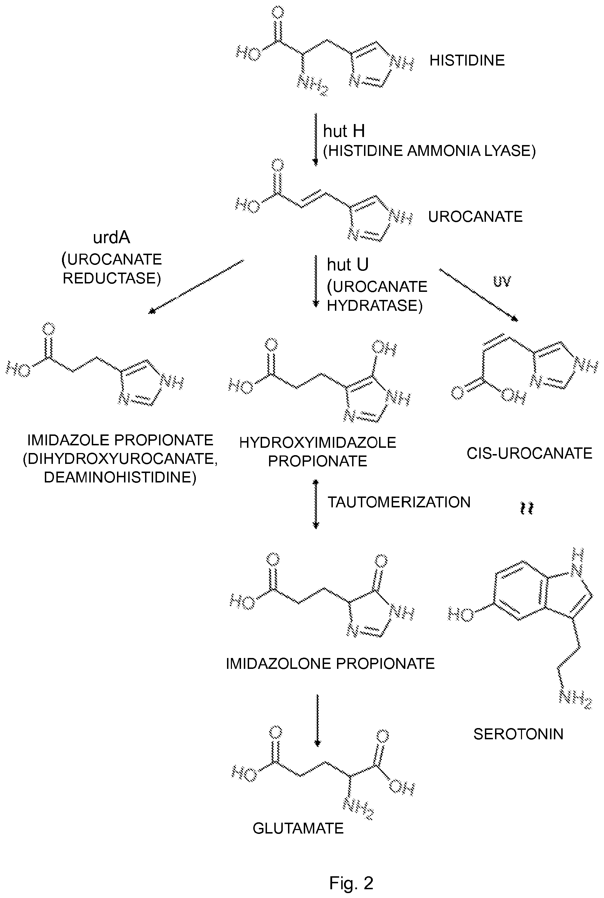

Fig. 2. Imidazole propionate as a histidine degradation product. The figure shows the histidine degradation pathway.

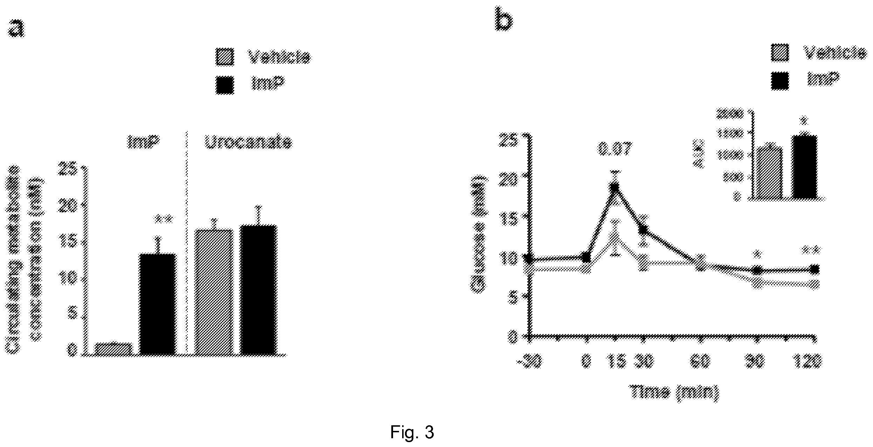

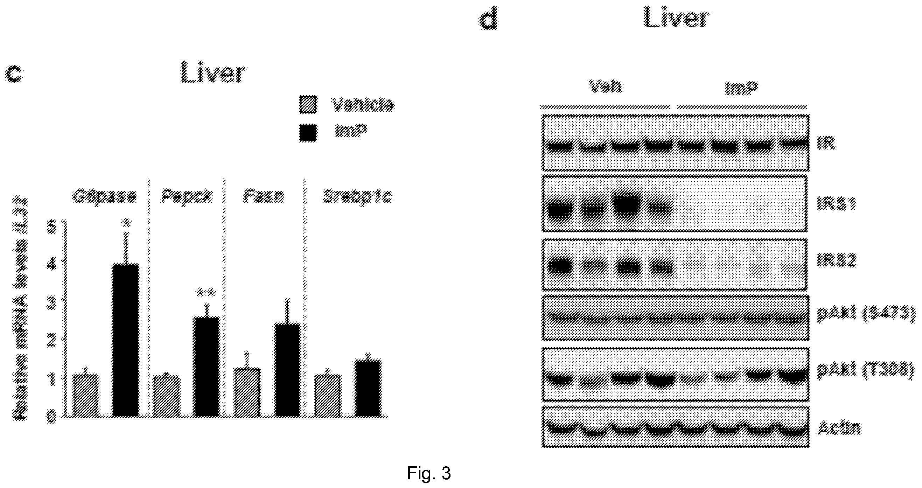

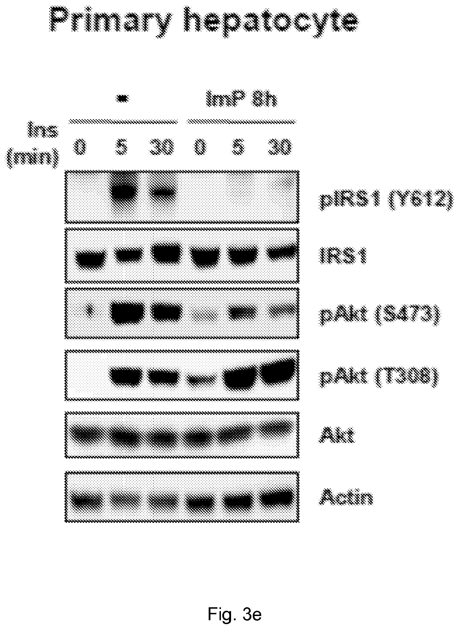

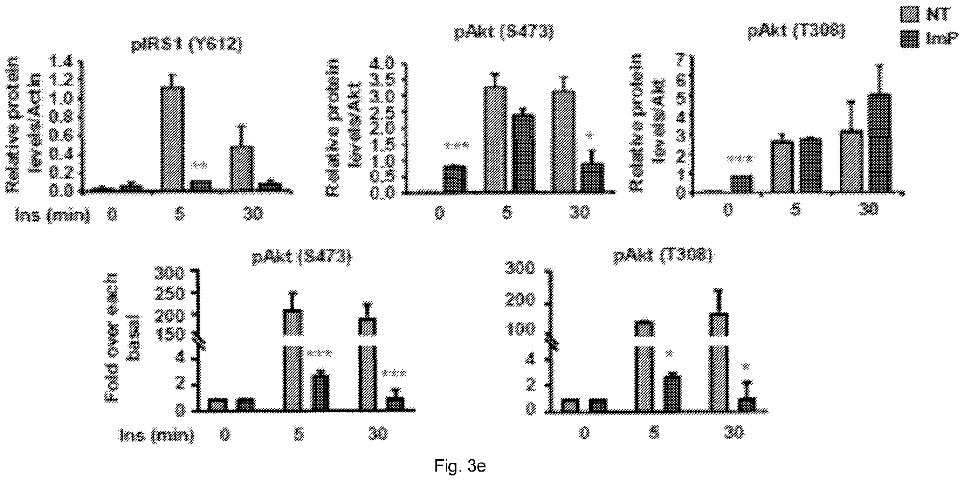

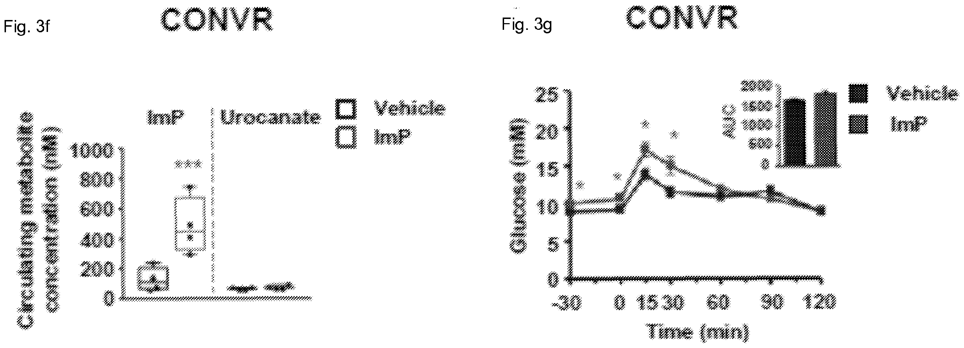

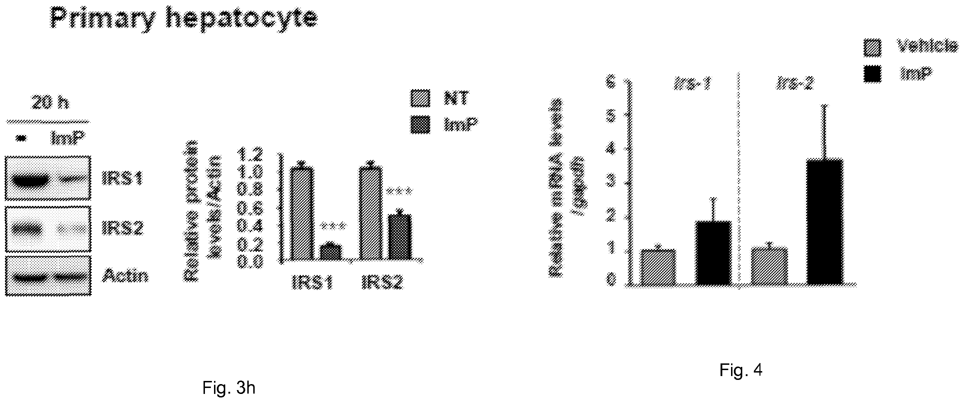

Fig. 3. Imidazole propionate impairs glucose tolerance and insulin signaling. Fig. 3a, Circulating levels of imidazole propionate (ImP) and urocanate, Fig. 3b, Intraperitoneal glucose tolerance test, and Fig. 3c, Expression of genes involved in gluconeogenesis and lipogenesis in GF mice after intraperitoneal injection of vehicle (1 % DMSO in water; n = 4) or ImP (500 µg; n = 5) twice per day for 3 days. Fig. 3d, Immunoblot of liver lysates from GF mice showing effect of 3-day treatment with vehicle or ImP (500 µg twice per day) on insulin signaling components. Fig. 3e, Effect of ImP (100 µM for 8 h) on insulin-stimulated IRS tyrosine phosphorylation and Akt activation [basal and insulin (5 nM) stimulated] in primary hepatocytes. Fig. 3f, Plasma levels of ImP and urocanate in CONVR mice after intraperitoneal injection of vehicle (1% DMSO in water; n = 4) or ImP (100 µg; n = 4) twice per day for 3 days. Fig. 3g, Intraperitoneal glucose tolerance test in CONVR mice injected with vehicle (n = 6) or ImP (100 µg; n = 5). Fig. 3h, Effect of ImP (100 µM for 20 h) on IRS protein levels in primary hepatocytes (n = 7). Data in Fig. 3a-3c are mean ± s.e.m. **P* < 0.05, ***P* < 0.01. Unpaired two-tailed Student's t-tests. Data in Fig. 3f is presented as box plots and show minimum, 25% quartile, median, 75% quartile and maximum. Two-way ANOVA with repeated measurements followed by Sidak's multiple comparisons test (Fig. 3f) and unpaired two-tailed Student's tests (Figs. 3e, 3g, 3h)
Fig. 4. Imidazole propionate does not affect mRNA levels of *Irs* in GF mice. mRNA levels of *Irs-1* and *Irs-*2 in the liver of GF mice after intraperitoneal injection of vehicle (1 % DMSO in water; n = 4) or imidazole propionate (ImP) (500 µg; n = 5) twice per day for 3 days.

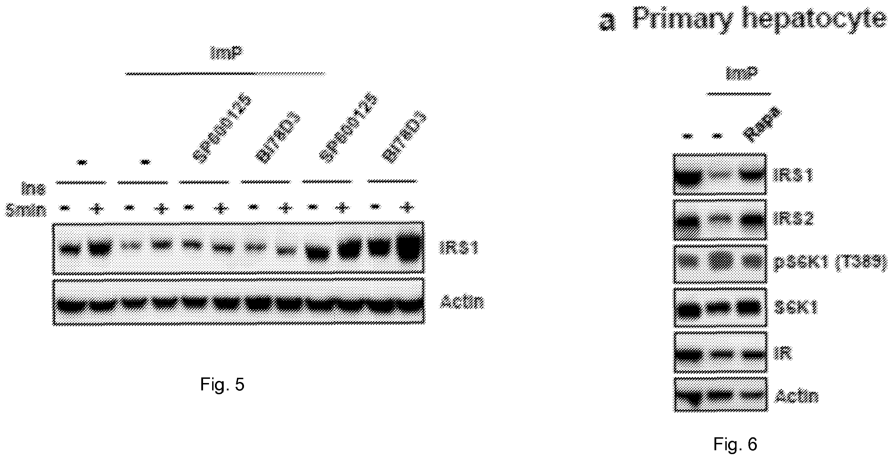

Fig. 5. JNK-independent IRS reduction by imidazole propionate. Primary hepatocytes were incubated with ImP (100 µM) in the absence or presence of the JNK inhibitors SP600125 (20 µM) or BI78D3 (20 µM) for 24 h and then stimulated with insulin (5 nM) for 5 min.

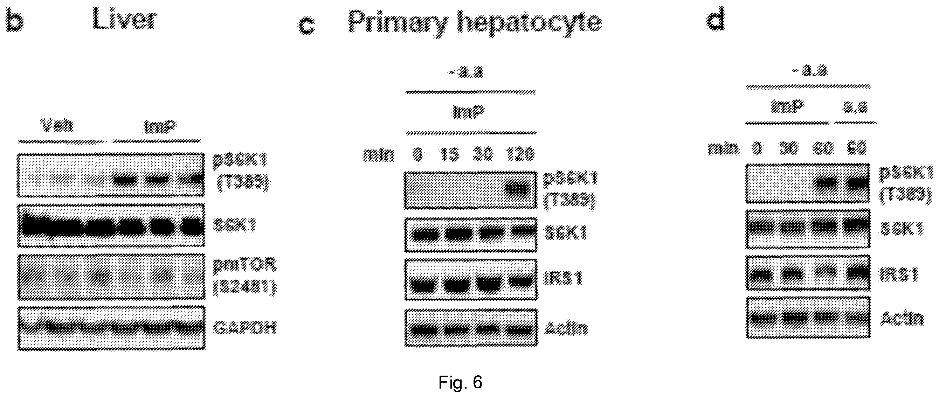

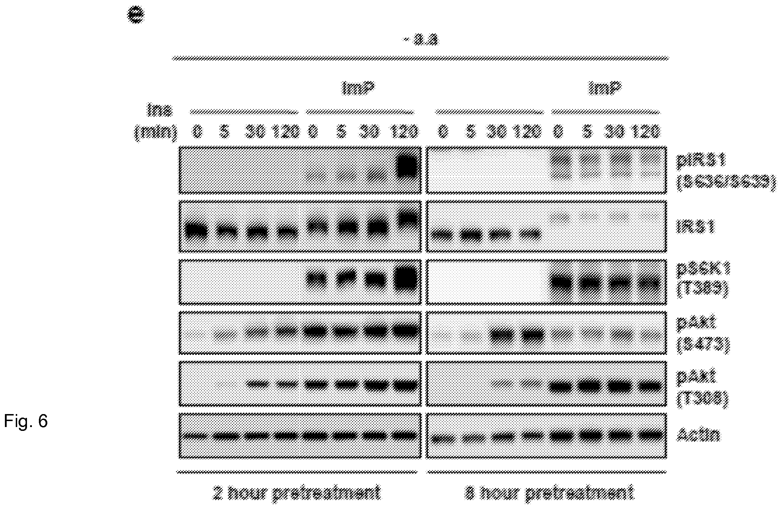

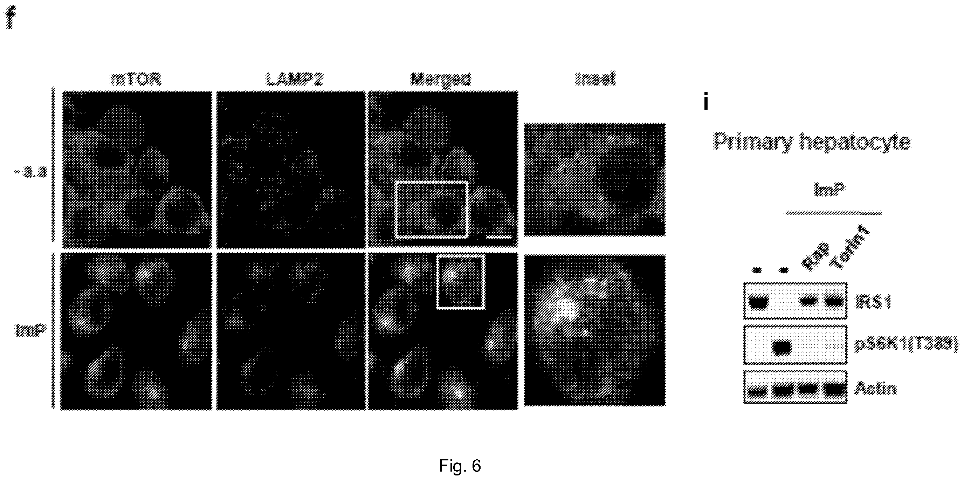

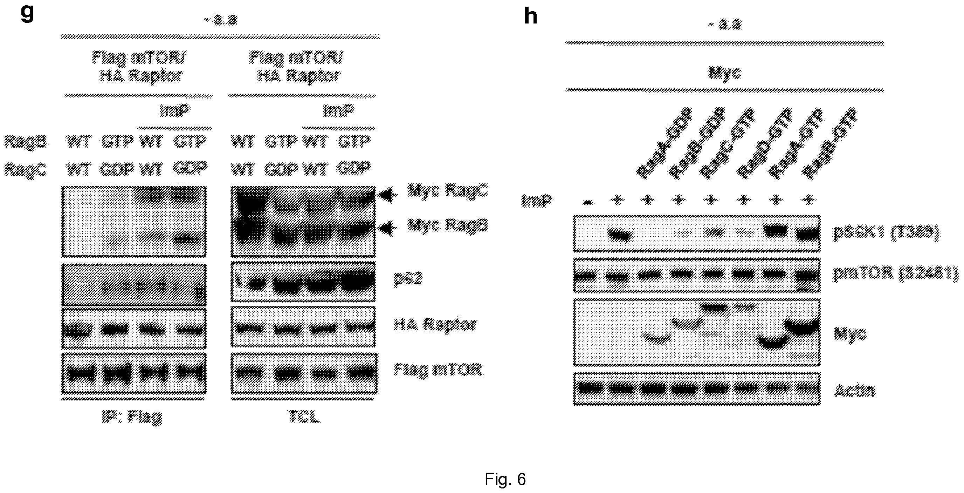

Fig. 6. Imidazole propionate activates mTORC1 via the amino acid sensing pathway at the lysosome. Fig. 6a, Rapamycin (20 nM) inhibits the effect of imidazole propionate (ImP) (100 µM for 24 h) on IRS and pS6K1 in primary hepatocytes. Fig. 6b, Immunoblot of liver lysates from GF mice showing that 3-day treatment with vehicle or ImP (500 µg twice per day) increases phosphorylation pf pS6K1 but not mTOR S2481. Figs. 6c, 6d, Rapid effects of ImP (100 µM) on pS6K1 in amino acid-deprived primary hepatocytes (Fig. 6c) and HEK293 cells (Fig. 6d). Fig. 6e, Effect of 2 and 8 h pretreatment with ImP (100 µM) on insulin signaling components in amino acid-deprived HEK293 cells incubated with insulin (5 nM) for the indicated times. Fig. 6f, Images of amino acid-deprived HEK293 cells incubated with ImP (100 µM for 1 h) and co-immunostained for mTOR and LAMP2, showing lysosomal localization of mTOR. Scale bar, 10 µm. Fig. 6g, ImP induced active p62/Rag/GTPases/mTORC1 complex formation. HEK293 cells were co-transfected with FLAG mTOR, HA Raptor and Myc Rag GTPases for 24 h and incubated with ImP (100 µM) in the absence of amino acids for 1 h. IP: immunoprecipitate, TCL total cell lysate. Fig. 6h, Involvement of Rag GTPases in ImP-induced mTORC1 activation. HEK293 cells were transfected with Myc-tagged constructs as indicated for 24 h and incubated with 100 µM ImP in the absence of amino acids for 1 h. Fig. 6i, Rapamycin (20 nM) or Torin 1 (50 nM) inhibits the effect of imidazole propionate (ImP) (100 µM for 15 h) on IRS and pS6K1 in primary hepatocytes (n = 2).

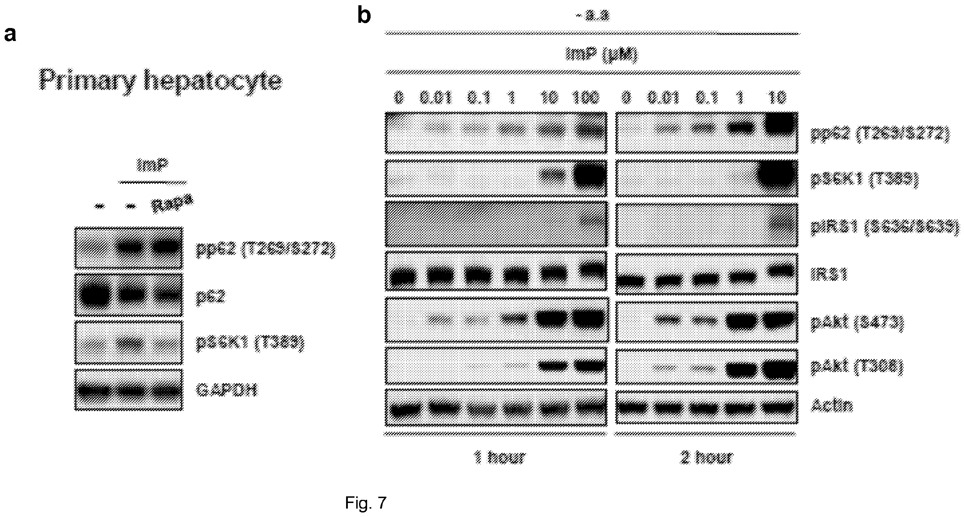

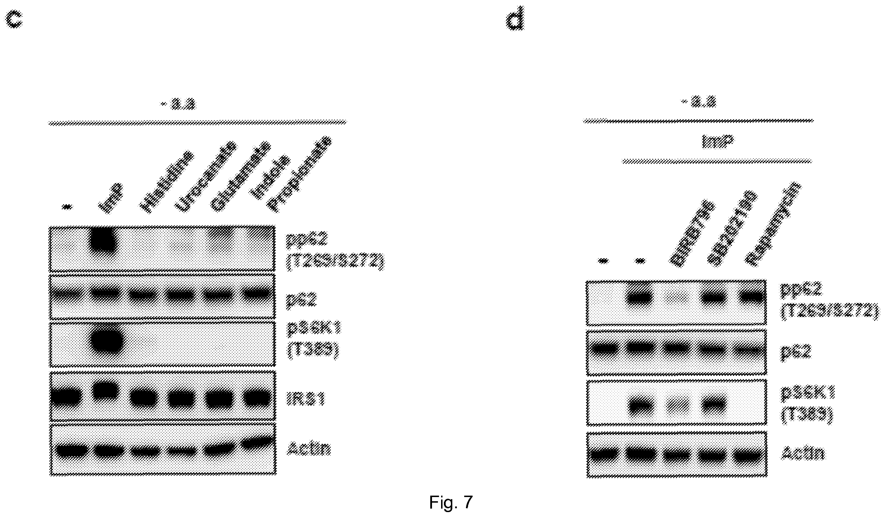

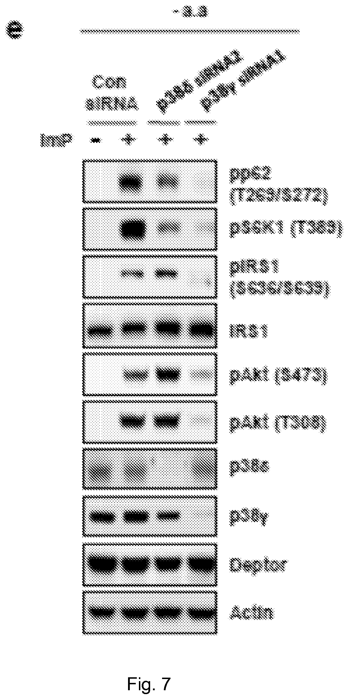

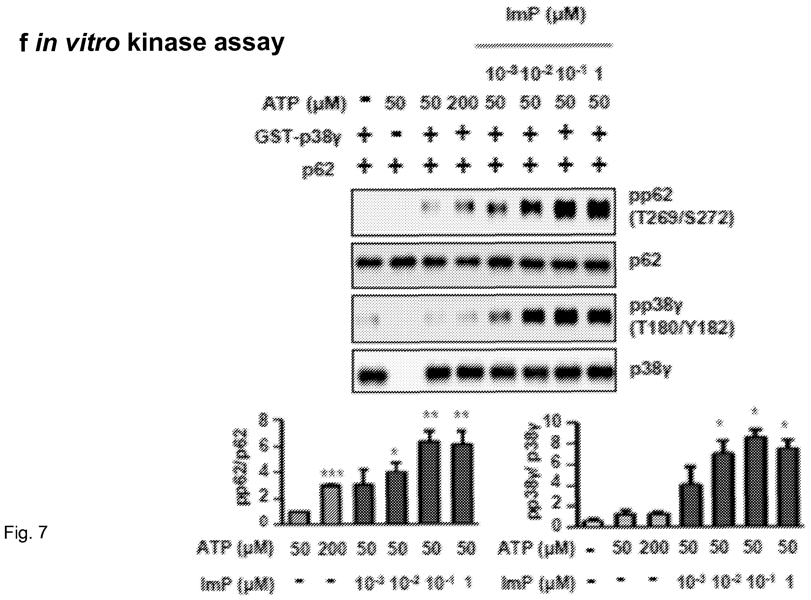

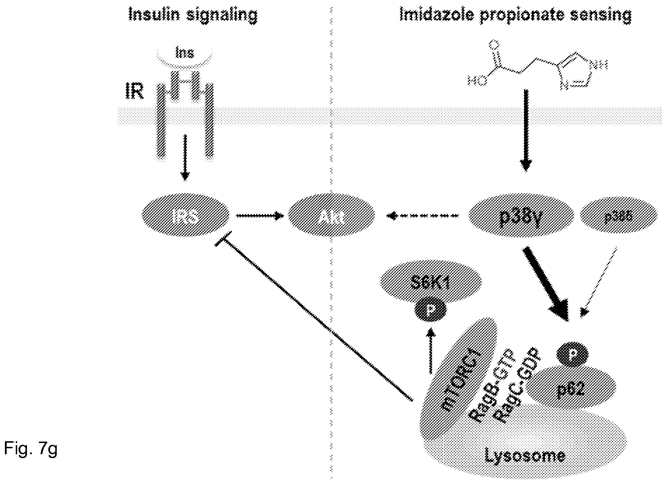

Fig 7. Imidazole propionate-mediated signaling is dependent on alternative p38 MAPK. Fig. 7a, Effect of imidazole propionate (ImP) (100 µM) and rapamycin (20 nM) for 24 h on p62 phosphorylation (T269 and S272) in primary hepatocytes. Fig. 7b, Effect of 1 and 2 h pretreatment with ImP (at the indicated concentrations) on insulin signaling components in amino acid-deprived HEK293 cells. Fig. 7c, Effect of ImP, histidine, urocanate, glutamate or indole propionate (100 µM for 1 h) on p62 phosphorylation (T269 and S272) and IRS1 in amino acid-deprived HEK293 cells. Fig. 7d, Effect of 30 min pretreatment with BIRB796 (10 µm), SB202190 (10 µM) or rapamycin (100 nM) on phosphorylation of p62 (T269 and S272) and S6K1 in amino acid-deprived HEK293 cells incubated with ImP (100 µM for 1 h). Fig. 7e, Effect of alternative p38 knockdown on ImP-induced signaling. HEK293 cells were transfected with control siRNA (con siRNA), p38δ siRNA2 or p38y siRNA1 for 24 h and incubated with ImP (100 µM) in the absence of amino acids for 1 h. Fig. 7f, *In vitro* kinase assay. p38y and p62 were preincubated and the kinase reaction was started by adding ATP in the absence or presence of ImP. Data are mean ± s.e.m. **P* < 0.05, ***P* < 0.01, ****P* < 0.001 vs ATP (50 µM) without ImP. Unpaired two-tailed Student's t-tests. Fig. 7g, Schematic depiction of ImP-induced signaling.
Fig, 8. Role of p38y in ImP-mediated signaling. HEK293 cells were transfected with control siRNA (con siRNA), p38δ siRNA or p38y siRNA for 24 h and incubated with ImP (100 µM) in the absence of amino acids for 1 h. Fig. 8a, Effects of two distinct nonoverlapping siRNAs against p38δ on ImP-induced mTORC1 activation. Fig. 8b, Effects of two distinct nonoverlapping siRNAs against p38γ on ImP-induced signaling.

### DETAILED DESCRIPTION

The present embodiments generally relate to metabolic disorders, and in particular to type 2 diabetes (T2D) and impaired glucose tolerance (IGT) and to diagnosing and treating such metabolic disorders.

The present embodiments are based on an association between T2D and IGT and an altered gut microbiota, which may contribute to the development of these diseases. In particular, microbially upregulated histidine-derived metabolite imidazole propionate is present at higher concentrations in subjects with T2D and IGT as compared to healthy subjects with normal glucose tolerance (NGT). The microbially produced imidazole propionate reduces insulin receptor substrate (IRS) protein levels and impairs insulin signaling through the alternative p38 mitogen-activated protein kinase (MAPK) activation pathway of mechanistic target of rapacymin complex 1 (mTORC1). Thus, the microbial metabolite imidazole propionate contributes to the pathogenesis of T2D and IGT.

Impaired glucose tolerance (IGT) is a pre-diabetic state of hyperglycemia that is associated with insulin resistance and increased risk of cardiovascular pathology. IGT may precede T2D by many years. According to the criteria of the World Health Organization (WHO) and the American Diabetes Association (AAD), IGT is defined as two-hour glucose levels of 140 to 199 mg per dl, i.e., 7.8 to 11.0 mmol/l, on the 75-g oral glucose tolerance test. A patient is said to be under the condition of IGT when he/she has an intermediately raised glucose level after 2 hours, but less than the level that would qualify for T2D. The fasting glucose may be either normal or mildly elevated, typically less than 6.1 mmol/l.

Type 2 diabetes (T2D), also referred to as type 2 diabetes mellitus, is a long term metabolic disorder that is characterized by high blood sugar, insulin resistance, and relative lack of insulin. Long-term complications from high blood sugar include heart disease; stroke; diabetic retinopathy, which can result in blindness; kidney failure; and poor blood flow in the limbs which may lead to amputations.

The WHO definition of T2D is for a single raised glucose reading with symptoms, otherwise raised values on two occasions, of either fasting plasma glucose ≥ 7.0 mmol/l (126 mg/dl) or with a glucose tolerance test, two hours after the oral dose a plasma glucose ≥ 11.1 mmol/l (200 mg/dl).

Table 1 below summaries the WHO diabetes diagnostic criteria.

**Table 1 - WHO diabetes diagnostic criteria**

| **Condition** | **2 hour glucose mmol/l (mg/dl)** | **Fasting** glucose **mmol/l (mg/dl)** | **HbA_{1c}** | |
|---|---|---|---|---|
| | | | **mmol/mol** | **DCCT %** |
| Normal | <7.8 (<140) | <6.1 (<110) | <42 | <6.0 |
| IFG | <7.8 (<140) | ≥6.1 (≥110) & <7.0 (<126) | 42-46 | 6.0-6.4 |
| IGT | ≥7.8 (≥140) | <7.0 (<126) | 42-46 | 6.0-6.4 |
| T2D | ≥11.1 (≥200) | ≥70 (≥126) | ≥48 | ≥6.5 |

| | | | | |
|---|---|---|---|---|
| IFG - impaired fasting glycemia HbA_{1c} - glycated hemoglobin DCCT - Diabetes Control and Complications Trial | | | | |

An aspect of the embodiments relates to a method of determining whether a subject is suffering from, or having a risk of suffering from, T2D or IGT. The method comprises determining an amount of imidazole propionate in a body sample from the subject. The method also comprises determining whether the subject is suffering from, or having a risk of suffering from, T2D or IGT based on the determined amount of imidazole propionate.

In this embodiment, the amount or concentration of imidazole propionate as determined in a body sample from the subject is used to determine whether the subject is suffering from, or at least having a risk of suffering from, T2D or IGT. As is further shown herein, subjects suffering from IGT or T2D have significantly higher amounts and concentrations of imidazole propionate as compared to normal, i.e., healthy, subjects and also as compared to IFG subjects.

Imidazole propionate is thereby, in this embodiment, used as a biomarker in order to identify individuals that may suffer from T2D or IGT or are likely to suffer from, i.e., having a risk of suffering from, these metabolic disorders.

The subject is a mammal subject, and preferably a human subject. However, the embodiments may also be applied to other mammal subjects that may suffer from T2D or IGT. Thus, the embodiments can also be used for veterinary purposes in order to identify animal subjects that are suffering from, or having a risk of suffering from, T2D or IGT.

In an embodiment, the method is a so-called decision support method, i.e., a non-diagnostic method. This means that the decision support method will generate decision support information, e.g., as exemplified by an amount or concentration of imidazole propionate, which is merely interim results. Additional data and the competence of a physician or veterinary are typically required for providing a final diagnosis. Furthermore, other parameters than the amount of imidazole propionate may influence the progression or development of T2D or IGT, such as the case history, age and sex of the subject, genetic factors etc. Thus, the embodiment gives a decision support upon, which a physician or veterinary can base his/her decision about which measures that should be taken.

In such an embodiment, a method of generating decision support information is provided. The method comprises generating the decision support information based on an amount of imidazole propionate determined in a body sample from the subject.

In an embodiment, the amount of imidazole propionate is determined in a body fluid sample from the subject. Illustrative, but non-limiting, examples of such body fluid samples include a blood sample, a plasma sample, a serum sample, a urine sample and a fecal sample. In a particular embodiment, the body sample fluid is selected from the group consisting of a blood sample, a plasma sample and a serum sample.

The amount of imidazole propionate in the body sample can be determined according to various embodiments. In a particular embodiment, the amount of imidazole propionate in the body sample is determined using mass spectrometric (MS) analysis. For instance, the body fluid sample could be extracted with ice-cold acetonitrile and dried under nitrogen flow. The body fluid sample may then be reconstituted with HCI, such as 5 % HCI, in n-butanol at, for instance, 70°C for 1 hour, allowing n-butyl ester to be formed. After this derivatization, the sample may be evaporated and reconstituted in water:acetonitrile, such as 90:10. Samples may then be injected onto a column, such as C18 ethylene bridged hybrid (BEH) column, using a gradient, such as a gradient consisting of water with, for instance, 0.1 % formic acid as A-phase and acetonitrile with, for instance, 0.1 % formic acid as B-phase. Imidazole propionate may then be detected by multiple reaction monitoring using the transitions 197/81.

The embodiments are, however, not limited to MS-based determination of imidazole propionate in the body sample. For instance, competitive enzyme-linked immunosorbent assay (ELISA) using tracers with imidazole propionate, such as imidazole propionate-acetylcholinesterase (AChE) conjugate or biotinylated imidazole propionate, which can be detected by using, for instance, Ellman's Reagent or horseradish peroxidase conjugated streptavidin with 3,3',5,5'-tetramethylbenzidine (TMB) as substrate, respectively. In such a case, the intensity of color after reaction will be inversely proportional to the amount of imidazole propionate in the body sample. In the competitive ELISA, a labelled imidazole propionate is thereby used as tracer.

Actually, any method or technology that can be used to directly, or indirectly, determine or at least estimate the amount or concentration of imidazole propionate in the body fluid sample can be used according to the embodiments.

In an embodiment, a concentration of imidazole propionate is determined in the body sample. The concentration of imidazole propionate is then compared with a threshold concentration and the subject is determined to suffer from, or having a risk of suffering from, T2D or IGT if the concentration of imidazole propionate is equal to or larger than the threshold value.

In an embodiment, the threshold concentration is equal to 5 nM, such as 5.1 nM. The threshold concentration is preferably 6 nM, such as 7 nM, e.g, 7.7 nM or 7.8 nM. In a particular embodiment, the threshold concentration is 8 nM, preferably 9 nM, and more preferably 10 nM, such as 10.1 nM.

Experimental data indicates that subjects with normal glucose tolerance (NGT) had an average imidazole propionate concentration of 9.1 nM (median 7.1 nM), subjects with an impaired fasting glucose (IFG) had an average imidazole propionate concentration of 9.1 mM (median 7.2 nM), IGT subjects had an average imidazole propionate concentration of 10.5 nM (median 7.7 nM), whereas T2D subjects had an average imidazole propionate concentration of 20.1 nM (median 13.8 nM). Furthermore, 75 % of the NGT subjects had an imidazole propionate concentration of less than 10.7 nM, 75 % of the IFG subjects had an imidazole propionate concentration of less than 11.7 nM, 75 % of the IGT subjects had an imidazole propionate concentration of less than 13.1 nM, and 75 % of the T2D subjects had an imdiazole propionate concentration of less than 19.1 nM.

In a particular embodiment, the method is a method of determining whether a subject is suffering from, or having a risk of suffering from, T2D. The method comprises determining an amount of imidazole propionate in a body sample from the subject. The method also comprises determining whether the subject is suffering from, or having a risk of suffering from, T2D based on the determined amount of imidazole propionate.

The imidazole propionate determined or measured according to the embodiments is preferably microbe-derived imidazole propionate and in particular bacteria-derived imidazole propionate.

If the concentration of the imidazole propionate is below the threshold concentration, then the subject is preferably determined or identified as not suffering from T2D or IGT, or having a low risk of suffering from of T2D or IGT.

In an embodiment, the concentration of imidazole propionate is compared with an IGT threshold concentration and with a T2D threshold concentration. In such a case, the method comprises determining that the subject is suffering from, or having a risk of suffering from, IGT if the concentration of imidazole propionate is equal to or larger than the IGT threshold concentration but lower than the T2D threshold concentration. Correspondingly, the method also comprises determining that the subject is suffering from, or having a risk of suffering from, T2D if the concentration of imidazole propionate is equal to or larger than the T2D threshold concentration.

In this embodiment, the IGT threshold concentration is lower than the T2D threshold concentration.

In an embodiment, the IGT threshold concentration is equal to 5 nM, such as 5.1 nM. The IGT threshold concentration is preferably 6 nM, such as 7 nM, e.g, 7.7 nM. In a particular embodiment, the IGT threshold concentration is 8 nM, preferably 9 nM, and more preferably 10 nM.

In an embodiment, the IGT threshold concentration is preferably equal to or larger than 7.2 nM, such as equal to or larger than 9.1 nM.

In an embodiment, the T2D threshold concentration is equal to 7 nM, such as 7.8 nM. The T2D threshold concentration is preferably 8 nM, such as 9 nM. In a particular embodiment, the T2D threshold concentration is 10 nM, such as 10.1 nM, preferably 11 nM, and more preferably 12 nM, with the proviso that the IGT threshold concentration < the T2D threshold concentration.

In an embodiment, the T2D threshold concentration is above 9.1 nM but preferably below 20.1 nM. More preferably, the T2D threshold concentration is above 10.7 nM, and preferably below 19.1 nM, such as below 13.8 nM. Hence, the T2D threshold concentration is preferably selected within an interval of from 10.7 up to 13.8 nM, such as 10.75 nM, 11 nM, 11.25 nM, 11.5 nM, 11.75 nM, 12 nM, 12.25 nM, 12.5 nM, 12.75 nM, 13 nM, 13.25 nM, 13.5 nM or 13.75 nM.

In an embodiment, not only the amount of imidazole propionate is measured in the body sample but also the amount of urocanate. In such an embodiment, the determination of whether the subject is suffering from or having a risk of suffering from T2D or IGT is based on the amount of imidazole propionate and the amount of urocanate, preferably based on a ratio or quotient between the amount of imidazole propionate and the amount of urocanate.

Hence in this embodiment the method also comprises determining an amount of urocanate in the body sample. The method also comprises determining whether the subject is suffering from, or having a risk of suffering from, T2D or IGT based on ratio between the determined amount of imidazole propionate and the determined amount of urocanate.

Experimental data indicates that subjects with NGT had an average imidazole propionate to urocanate (ImP/Uro) ratio of 0.1433, IFG subjects had an average ImP/Uro ratio of 0.1335, IGT subjects had an average ImP/Uro ratio of 0.1657 and T2D subjects had an average ImP/Uro ratio of 0.3417. Furthermore, 75 % of the NGT subjects had an ImP/Uro ratio of less than 0.1677, 75 % of the IFG subjects had an ImP/Uro ratio of less than 0.1644, and 75 % of the IGT subjects had an ImP/Uro ratio of less than 0.1836.

Accordingly, an ImP/Uro ratio threshold selected within an interval of 0.15 and 0.325 could be used to determine whether the subject is suffering from or having a risk of suffering from T2D or IGT.

In a particular embodiment, an ImP/Uro ratio threshold is selected within an interval of 0.17 and 0.3, preferably within an interval of 0.2 and 0.3, such as 0.2, 0.21, 0.22, 0.23, 0.24, 0.25, 0.26, 0.27, 0.28, 0.29 or 0.3. An ImP/Uro ratio threshold within 0.2 and 0.3 is in particular preferred when determining whether the subject is suffering from, or having a risk of suffering from, T2D.

The above presented values of ratio threshold and threshold values are in particular applicable if the body sample is a body fluid sample, such as a blood sample, a plasma sample or a serum sample.

Corresponding values of ImP/Uro ratio obtained from batch culture experiments using feces from obese subjects without T2D and from obese subjects with T2D are an average ImP/Uro ratio of 32.1152 for obese subjects without T2D and an average ImP/Uro ratio of 413.0492 for obese subjects with T2D. Correspondingly, 75 % of the obese non-T2D subjects had an ImP/Uro ratio less than 1.0255, whereas 75 % of the obese T2D subjects had an ImP/Uro ratio of less than 615.6992.

Accordingly, a suitable ImP/Uro ratio threshold for batch culture experiments using feces could be selected within an interval of 50 and 400, preferably within an interval of 100 and 400, more preferably within an interval of 200 and 400, such as within an interval of 200 and 300.

The above presented examples of ImP/Uro ratio thresholds are based on calculating a ratio or quotient between the determined amount of imidazole propionate and the determined amount of urocanate. If the ratio or quotient is instead between the determined amount of urocanate and the determined amount of imidazole propionate, i.e., Uro/ImP, then suitable Uro/ImP ratio thresholds can be obtained as one divided by the above presented values.

In an embodiment, the ratio between the determined amount of imidazole propionate and the determined amount of urocanate is compared to the ratio threshold and the subject is determined to suffer from, or having a risk of suffering from, T2D or IGT if the ratio between the determined amount of imidazole propionate and the determined amount of urocanate is equal to or larger than the threshold ratio.

This embodiment is equivalent to calculating a ratio between the determined amount of urocanate and the determined amount of imidazole propionate and comparing the calculated ratio with an Uro/ImP threshold ratio. In such a case, the subject is determined to suffer from, or having a risk of suffering from, T2D or IGT if the calculated ratio is equal to or smaller, such as smaller, than the Uro/ImP threshold ratio.

The above described embodiments of determining not only the amount of imidazole propionate but also the amount of urocanate can be applied to the previously described embodiments of a decision support method, i.e., a non-diagnostic method.

A particular embodiment relates to a method of determining whether a subject has or is at risk of developing T2D or IGT. The method comprises measuring an amount of imidazole propionate in a body sample from the subject. The method also comprises determining that the subject has or is at a risk of developing IGT if the concentration of imidazole propionate is equal to or greater than a threshold concentration for IGT but less than a threshold concentration for T2D and/or determining that the subject has or is at a risk of developing T2D if the concentration of imidazole propionate is equal to or greater than the threshold concentration for T2D.

In an example of this particular embodiment, the threshold concentration of imidazole propionate for IGT is 8 nM, preferably 9 nM, and more preferably 10 nM and the threshold concentration of imidazole propionate for T2D 10 nM, preferably 11 nM, and more preferably 12 nM, such as within an interval of from 10.7 nM up to 13.8 nM.

In a particular embodiment, the method further comprises treating the subject determined to have or be at risk of developing T2D or IGT. This treatment of the subject preferably comprises administering to the subject an effective amount of an inhibitor selected from the group consisting of an urocanate reductase inhibitor, a histidine ammonia lyase inhibitor, an inhibitor of an imidazole propionate producing bacteria, an inhibitor of p38y MAPK, an inhibitor of p38δ MAPK, an inhibitor of mTORC1, an inhibitor of alternative p38 MAPK activation pathway, and any combination thereof, and/or or an effective amount of an imidazole propionate derivative.

Imidazole propionate can directly modulate the p38 MAPK activity. Thus, imidazole propionate can be used as a scaffold to identify inhibitors for p38 MAPK. Accordingly, a dual targeting of both bacteria and host targets of imidazole propionate is possible. Potential inhibitors targeting urocanate reductase in bacteria are urocanate or imidazole propionate derivatives. Imidazole propionate can directly promote p38y kinase activity. Thus, structural analogues to imidazole propionate can potentially inhibit p38y gamma in a competitive way.

A further particular embodiment relates to a method of diagnosing a subject as having or being at risk of developing T2D or IGT. The method comprises measuring an amount of imidazole propionate in a body sample from the subject. In this particular embodiment, an amount of imidazole propionate of at least 8 nM, preferably 9 nM, and more preferably 10 nM, but less than 10 nM, preferably 11 nM, and more preferably 12 nM, diagnoses the subject as having or being at risk of developing IGT and an amount of imidazole propionate of at least 10 nM, preferably 11 nM, and more preferably 12 nM, diagnoses the subject as having or being at risk of developing T2D, thereby diagnosing a subject as having or being at risk of developing T2D.

In a particular embodiment, the subject is diagnosed as having or being at a risk of developing IGT if the amount of imidazole propionate is above an IGT concentration threshold within an interval of from 10.7 nM up to 13.8 nM.

In a particular embodiment, the method further comprises treating a subject determined to have or be at risk of developing T2D or IGT. Treating the subject preferably comprises administering to the subject an effective amount of an inhibitor selected from the group consisting of an urocanate reductase inhibitor, a histidine ammonia lyase inhibitor, an inhibitor of an imidazole propionate producing bacteria, an inhibitor of p38y MAPK, an inhibitor of p38δ MAPK, an inhibitor of alternative p38 MAPK activation pathway, an inhibitor of mTORC1, and any combination thereof, and/or or an effective amount of an imidazole propionate derivative.

Yet another particular embodiment relates to a method of determining whether a subject has or is at risk of developing T2D or IGT. The method comprises determining and/or measuring an amount of imidazole propionate in a body sample from the subject using a metabolite detecting device that is configured to provide a data output corresponding the amount of imidazole propionate in the body sample. The method also comprises determining whether the subject has or is at risk of developing T2D or IGT based on the data output.

In a particular embodiment, the metabolite detecting device is selected from a mass spectrometer, a spectrometer, such a spectrometer with ELISA, a liquid chromatography device, a gas chromatography device, a gas-liquid chromatography device, a high performance liquid chromatography device and any combination thereof, such as a combination of a mass spectrometer and chromatography device.

The metabolite detector device is, in an embodiment, also configured to provide a data output corresponding to the amount of uraconate in the body sample. Alternatively, a first metabolite detector is configured to provide a data output corresponding the amount of imidazole propionate in the body sample and a second metabolite detector is configured to provide a data output corresponding to the amount of uraconate in the body sample.

An embodiment relates to a method of determining whether a subject is suffering from, or having a risk of suffering from, T2D or IGT. The method comprises determining an amount of imidazole propionate producing bacteria in a body sample from the subject. The method also comprises determining whether the subject is suffering from, or having a risk of suffering from, type 2 diabetes or impaired glucose tolerance based on the determined amount of imidazole propionate producing bacteria.

In an embodiment, the imidazole propionate producing bacteria is selected from the group consisting of *Adlercreutzia, Aerococcus, Clostridium, Desulfatibacillum, Eggerthella, Enterobacter, Enterococcus, Enterococcus, Fusobacterium, Lactobacillus, Shewanella, and Streptococcus.* The method also comprises determining whether the subject has or is at risk of developing T2D or IGT based on the determined and/or measured amount of imidazole propionate producing bacteria in the body sample.

In an embodiment, the determination of whether the subject is suffering from, or having a risk of suffering from T2D or IGT could be based on detection of any ImP producing bacteria in the body sample. In another embodiment, the determination is based on a determined amount of ImP producing bacteria in the body sample. A further variant is to perform the determination based on the percentage of the bacteria in the body sample that are ImP producing bacteria.

Another aspect of the embodiments relates to a method of identifying an agent effective in preventing, inhibiting or treating T2D or IGT in a subject. The method comprises determining whether an agent is capable of inhibiting at least one of urocanate reductase, histidine ammonia lyase, p38y MAPK, p38δ MAPK and alternative p38 MAPK activation pathway. The method also comprises identifying the agent as effective in preventing, inhibiting or treating T2D or IGT in a subject if the agent is determined to be capable of inhibiting at least one urocanate reductase, histidine ammonia lyase, p38y MAPK, p38δ MAPK and alternative p38 MAPK activation pathway.

Urocanate reductase is an enzyme enriched in gut microbiota of subjects suffering from T2D and IGT. In these bacteria, the urocanate reductase, encoded by the gene *urdA,* is involved in the conversion of urocanate into imidazole propionate, see Fig. 2. This enzyme urocanate reductase is not, as far as the inventors are aware of, present in humans since no *urdA* homologs with more than 30 % sequence identity has been identified in the human genome.

Hence, in an embodiment, the method comprises determining whether the agent is capable of inhibiting urocanate reductase and identifying the agent as effective in preventing, inhibiting or treating T2D or IGT in the subject if the agent is determined to be capable of inhibiting urocanate reductase.

In a particular embodiment, the method comprises determining whether the agent is capable of inhibiting conversion of urocanate into imidazole propionate by urocanate reductase. In such a particular embodiment, the agent is identified as effective in preventing, inhibiting or treating T2D or IGT in said subject if said agent is determined to be capable of inhibiting conversion of urocanate into imidazole propionate by urocanate reductase.

The determination of whether the agent is capable of inhibiting urocanate reductase can be performed by measuring the amount of imidazole propionate produced given a challenge of urocanate in the presence and in the absence of the agent. In such a test, the enzyme may be in substantially pure form, such as recombinantly produced or isolated from the gut microbiota. Alternatively, the imidazole propionate producing bacteria could be challenged by urocanate and/or histidine in the presence or absence of the agent.

Histidine ammonia lyase is an enzyme involved in the conversion of histidine into urocanate. In humans, urocanate is further converted as shown in Fig. 2 into glutamate. In bacteria, urocanate reductase competes with urocanate hydratase in using urocanate as substrate. Inhibiting histidine ammonia lyase effectively reduces the amount of urocanate, thereby reducing the substrate of urocanate reductase. As a result of such inhibition of histidine ammonia lyase, the amount of imidazole propionate produced by bacteria will be significantly reduced.

Hence, in an embodiment, the method comprises determining whether the agent is capable of inhibiting histidine ammonia lyase and identifying the agent as effective in preventing, inhibiting or treating T2D or IGT in the subject if the agent is determined to be capable of inhibiting histidine ammonia lyase.

In a particular embodiment, the method comprises determining whether the agent is capable of inhibiting bacterial histidine ammonia lyase and identifying the agent as effective in preventing, inhibiting or treating T2D or IGT in said subject if the agent is determined to be capable of inhibiting bacterial histidine ammonia lyase.

Thus, in this particular embodiment the agent is preferably capable of inhibiting bacterial histidine ammonia lyase, encoded by the *hutH* gene, but is preferably not capable of inhibiting, or at least inhibiting but at lower inhibition level or degree, human histidine ammonia lyase, encoded by the *HAL* gene. In such an embodiment, the agent is capable of shutting down or at least reducing the pathway from histidine into imidazole propionate in bacteria but still allow, at least to some extent or degree, formation of glutamate in human cells from histidine.

In a particular embodiment, the agent is more effective in inhibiting bacterial histidine ammonia lyase as compared to human histidine ammonia lyase. In particular, the agent is preferably 1.25 times, 1.5 times, twice, three times, four times, five time or more effective in inhibiting bacterial histidine ammonia lyase as compared to human histidine ammonia lyase.

In another particular embodiment, the agent is a non-absorbable inhibitor, i.e., an inhibitor not absorbed by the gastrointestinal tract of the subject. This means that the inhibitor will be present in the gastrointestinal tract to thereby exert its inhibiting effect, such as on bacterial histidine ammonia lyase of the bacteria in the gastrointestinal tract. Non-absorbable as used herein does not necessarily implies that the inhibitor cannot be absorbed by the intestinal mucosa of the large intestine, e.g., the colon, and/or the small intestine, e.g., the ileum. A non-absorbable inhibitor as used herein also includes inhibitors having a significantly lower absorption by the intestinal mucosa as compared to agents that are readily absorbed by the large and/or small intestine. This means that the non-absorbable inhibitor may to some extent be absorbed by the intestinal mucosa but then at a lower extent or at a lower absorption kinetics as compared to readily absorbed agents.

The agent is preferably capable of inhibiting conversion of histidine into urocanate by histidine ammonia lyase, more preferably by bacterial histidine ammonia lyase. The agent is then identified as effective in preventing, inhibiting or treating type 2 diabetes or impaired glucose tolerance in the subject if the agent is determined to be capable of inhibiting conversion of histidine into urocanate by histidine ammonia lyase, more preferably by bacterial histidine ammonia lyase.

The determination of whether the agent is capable of inhibiting histidine ammonia lyase can be performed by measuring the amount of urocanate produced given a challenge of histidine in the presence and in the absence of the agent. In such a test, the enzyme may be in substantially pure form, such as recombinantly produced or isolated from the gut microbiota. Alternatively, the imidazole propionate producing bacteria could be challenged by histidine in the presence or absence of the agent.

Fig. 7g is a schematic depiction of imidazole propionate-induced signaling. As is shown in this figure and further disclosed herein, imidazole propionate is capable of binding to and activating or inducing p38y MAPK and possibly p38δ MAPK. This activation of p38y and p38δ MAPK in turn starts an activation pathway that results in the degradation of IRS. This means that inhibition of p38y MAPK and/or p38δ MAPK would be an effective way of inhibiting the deleterious effects of imidazole propionate in subjects suffering from T2D or IGT.

Accordingly, in an embodiment, the method comprises determining whether the agent is capable of inhibiting p38y MAPK and identifying the agent as effective in preventing, inhibiting or treating T2D or IGT in the subject if the agent is determined to be capable of inhibiting p38y MAPK.

In a particular embodiment, the method comprises determining whether the agent is capable of inhibiting p38y MAPK in phosphorylating at least one of sequestosome-1 (p62), ribosomal protein S6 kinase beta-1 (S6K1), insulin receptor substrate 1 (IRS1), protein kinase B (PKB) and p38y MAPK. The method also comprises, in this particular embodiment, identifying the agent as effective in preventing, inhibiting or treating T2D or IGT in the subject if the agent is determined to be capable of inhibiting p38y MAPK in phosphorylating at least one of p62, S6K1, IRS1, PKB and p38y MAPK. p38y MAPK is capable of phosphorylating itself in an autophosphorylation process.

The determination of whether the agent is capable of inhibiting p38y MAPK could be determined as further described herein to using antibodies against phospho-p62, against phospho-S6K1, against phospho-IRS1, and/or phospho-PKB and/or phospho-p38y MAPK. The amount of phosphorylated forms of these molecules in the presence and in the absence of the agent could then be determined.

In another embodiment, the method comprises determining whether the agent is capable of inhibiting p38δ MAPK and identifying the agent as effective in preventing, inhibiting or treating T2D or IGT in the subject if the agent is determined to be capable of inhibiting p38δ MAPK.

In a particular embodiment, the method comprises determining whether the agent is capable of inhibiting p38δ MAPK in phosphorylating at least one of p62, S6K1, IRS1, PKB and p38δγ MAPK. The method also comprises, in this particular embodiment, identifying the agent as effective in preventing, inhibiting or treating T2D or IGT in the subject if the agent is determined to be capable of inhibiting p38δ MAPK in phosphorylating at least one of p62, S6K1, IRS1, PKB and p38δγ MAPK.

The determination of whether the agent is capable of inhibiting p38δ MAPK could be determined as further described herein to using antibodies against phospho-p62, against phospho-S6K1, against phospho-IRS1, phospho-PKB, and/or phospho-p38δ MAPK. The amount of phosphorylated forms of these molecules in the presence and in the absence of the agent could then be determined.

Experimental data presented herein shows that imidazole propionate impairs insulin signaling by activating the alternative p38 MAPK pathway. This alternative p38 MAPK pathway involves activation of p38δ and/or p38y MAPK. This alternative p38 MAPK pathway is different from the regular p38 MAPK pathway that involves activation of p38α and p38β MAPK.

Hence, in an embodiment, the method comprises determining whether the agent is capable of inhibiting alternative p38 MAPK activation pathway and identifying the agent as effective in preventing, inhibiting or treating T2D or IGT in the subject if the agent is determined to be capable of inhibiting alternative p38 MAPK activation pathway.

Inhibition of the alternative p38 MAPK activation pathway can be assessed or determined by monitoring any molecule involved in the pathway from activation of p38δ and/or p38y MAPK to the final outcome of degradation of IRS as shown in Fig. 7g. For instance, phosphorylation of the targets of p38δ and/or p38y MAPK can be monitored in the presence and in the absence of the agent using antibodies as described herein.

Thus, various inhibitors could be identified as agents effective in preventing, inhibiting or treating T2D or IGT. The inhibitor may be an inhibitor of the production of imidazole propionate by bacteria in the gastrointestinal tract, such as an urocanate reductase inhibitor or a histidine ammonia lyase inhibitor. Alternatively, the inhibitor may be an inhibitor active in the signal pathway activated by imidazole propionate and shown in Fig. 7g, such as a p38y MAPK inhibitor, a p38δ MAPK inhibitor or an inhibitor of the alternative p38 MAPK pathway.

The above described embodiments of identifying the agent are preferably *in vitro* methods. Hence, the methods are preferably performed *in vitro,* such as by determining *in vitro* whether an agent is capable of inhibiting at least one of urocanate reductase, histidine ammonia lyase, p38y MAPK, p38δ MAPK and alternative p38 MAPK pathway.

In another aspect, the agent effective in preventing, inhibiting or treating T2D or IGT is identified using an *in vitro* simulated human, or other target mammal, intestinal model as further disclosed herein. Such an aspect thereby relates to a method of identifying an agent effective in preventing, inhibiting or treating T2D or IGT in a subject. The method comprises adding an agent to an *in vitro* simulated human intestinal model challenged with histidine and/or urocanate. An amount of imidazole propionate produced by the *in vitro* simulated human intestinal model is measured. The agent is then identified as effective in preventing, inhibiting or treating T2D or IGT based on the amount of imidazole propionate.

In an embodiment, the agent is added to an *in vitro* simulated human intestinal model inoculated with an aliquot of feces from a subject suffering from T2D of IGT. An example of such an *in vitro* simulated human intestinal model is further described in the example section.

In an embodiment, the method comprises comparing the amount of imidazole propionate with a reference amount of imidazole propionate produced by an *in vitro* reference simulated human intestinal model. The *in vitro* reference simulated human intestinal model lacks any urocanate reductase producing bacteria or is inoculated with an aliquot of feces from a subject with normal glucose tolerance. The agent is then identified as effective in preventing, inhibiting or treating T2D or IGT based a comparison of the amount of imidazole propionate and the reference amount of imidazole propionate.

Thus, in this embodiment a reference amount of imidazole propionate is determined and compared to the amount of imidazole propionate produced by the *in vitro* reference simulated human intestinal model challenged with histidine and/or urocanate and following addition of the agent. This *in vitro* reference simulated human intestinal model mimics the gastrointestinal tract of a healthy human subject, i.e., a subject that does not suffer from T2D or IGT. This means that if the agent is capable of reducing the amount of imidazole propionate produced by the *in vitro* simulated human intestinal model down to, or at least close to, the reference amount, then the agent is identified as effective in preventing, inhibiting or treating T2D or IGT.

In another embodiment, the method comprises comparing the amount of imidazole propionate with a reference amount of imidazole propionate produced by the *in vitro* simulated human intestinal model challenged with histidine and/or urocanate but in absence of the agent. The agent is then identified as effective in preventing, inhibiting or treating T2D or IGT based a comparison of the amount of imidazole propionate and the reference amount of imidazole propionate.

In an embodiment, not only the amount of imidazole propionate is measured but also the amount of urocanate. In such an embodiment, the method comprises measuring an amount of urocanate accumulated in the *in vitro* simulated human intestinal model. The method also comprises identifying the agent as effective in preventing, inhibiting or treating T2D or IGT based on the amount of imidazole propionate and the amount of urocanate, such as based on a ratio the amount of imidazole propionate and the amount of urocanate.

In an embodiment, the ratio between the amount of imidazole propionate and the amount of urocanate may be compared to a reference ratio calculated as the ratio between a reference amount of imidazole propionate and a reference amount of urocanate produced by the *in vitro* simulated human intestinal model challenged with histidine and/or urocanate but in the absence of the agent. The agent is then identified as effective in preventing, inhibiting or treating T2D or IGT based on a comparison of the ratio and the reference ratio.

In these embodiments, the reference amount of imidazole propionate or urocanate is the amount of imidazole propionate produced by or the amount of urocanate accumulated in the *in vitro* simulated intestinal model in the absence of the agent. This means that if the amount of imidazole propionate is significantly lower than the reference amount of imidazole propionate and/or the amount of urocanate is significantly higher than the reference amount of urocanate, then the agent is identified as effective in preventing, inhibiting or treating T2D or IGT based a comparison of the amount of imidazole propionate or urocanate and the reference amount of imidazole propionate or urocanate.

A further aspect of the embodiments relates to a method of preventing, inhibiting or treating T2D or IGT in a subject. The method comprises administering an effective amount of an inhibitor selected from the group consisting of an urocanate reductase inhibitor, a histidine ammonia lyase inhibitor, an inhibitor of an imidazole propionate producing bacteria, an inhibitor of p38y MAPK, an inhibitor of p38δ MAPK, an inhibitor of mTORC1 and an inhibitor of alternative p38 MAPK activation pathway to the subject.

In an embodiment, the method comprises administering an effective amount of an imidazole propionate (ImP) derivative or analogue. Such an imidazole propionate derivative has been shown to inhibit the ImP-induced induction of histidine ammonia lyase but it itself did not induce this enzyme. This means that such ImP derivative may block the conversion of histidine into urocanate and further into imidazole propionate.

A non-limiting, but illustrative, example of such an ImP derivative that could be used is chloroimadazole propionate.

In an embodiment, the inhibitor is an mTORC1 inhibitor. As is further shown in Fig. 7g and further described herein, such an mTORC1 inhibitor inhibits the ImP-induced reduction of IRS. Hence, inhibiting mTORC1 will effectively inhibit the ImP-induced degradation of IRS.

Non-limiting, but illustrative, examples of such mTORC1 inhibitors include rapamycin, also referred to as Sirolimus or (7E,15E,17E,19E)-9,10,12,13,14,21,22,23,24,25,26,27,32,33,34aS-Hexadecahydro-9R,27-dihydroxy-3S-[(1R)-2-[(1S,3R,4R)-4-hydroxy-3-methoxycyclohexyl]-1-methylethyl]-10R,21S-dimethoxy-6R,8,12R,14S,20,26R-hexamethyl-23S,27R-epoxy-3H-pyrido[2,1-c][1,4]oxaazacyclohentriacontine-1,5,11,28,29(4H,6H,31H)-pentone (CAS 53123-88-9); everolimus, also referred to as 42-O-(2-hydroxyethyl)-rapamycin (CAS 159351-69-6); AZD8055, also referred to as CGI-168 or 5-[2,4-bis[(3S)-3-methyl-4-morpholinyl]pyrido[2,3-d]pyrimidin-7-yl]-2-methoxy-benzenemethanol (CAS 1009298-09-2); temsirolimus, also referred to as CGI-779 or 42-[3-hydroxy-2-(hydroxymethyl)-2-methylpropanoate] rapamycin (CAS 162635-04-31); PP242, also referred to as torkinib or 2-[4-amino-1-(1-methylethyl)-1H-pyrazolo[3,4-d]pyrimidin-3-yl]-1 H-indol-5-ol (CAS 1092351-67-1); torin1, also referred to as 1-[4-[4-(1-oxopropyl)-1-piperazinyl]-3-(trifluoromethyl)phenyl]-9-(3-quinolinyl)-benzo[h]-1,6-naphthyridin-2(1H)-one (CAS 1222998-36-8); and WYE-125132, also referred to as N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methyl-urea (CAS 1144068-46-1).

In an embodiment, the inhibitor is a p38y MAPK inhibitor. As is further shown in Fig. 7g and further described herein, such a p38y MAPK inhibitor inhibits the ImP-induced phosphorylation of p62 and S6K1, which are upstreams events, see Fig. 7g, that ultimately lead to the degradation of IRS.

Non-limiting, but illustrative, examples of such p38y MAPK inhibitors include 1-(5-*tert*-butyl-2-*p*-tolyl-2H-pyrazol-3-yl)-3-[4-(2-morpholin-4-yl-ethoxy)naphthalen-1-yl]urea (BIRB796, CAS 285983-48-4), pirfenidone (also referred to as perfenidone or 5-methyl-1-phenyl-2(1H)-pyridinone, CAS 53179-13-8) and RNAi molecules, such as microRNA (miRNA) and small interfering RNA (siRNA).

In an embodiment, the inhibitor is a p38δ MAPK inhibitor. As is further shown in Fig. 7g and further described herein, such a p38δ MAPK inhibitor inhibits the ImP-induced phosphorylation of p62 and S6K1, which are upstreams events, see Fig. 7g, that ultimately lead to the degradation of IRS.

Non-limiting, but illustrative, examples of such p38δ MAPK inhibitors include BIRB796 and RNAi molecules, such as miRNA and siRNA.

In an embodiment, the inhibitor is an urocanate reductase inhibitor. The inhibitor could achieve its inhibiting effect by binding to and blocking or at least interfering with the enzymatic action of urocanate reductase. Alternatively, the urocanate reductase inhibitor could inhibit or at least reduce the transcription, translation and/or any post-translational processing of urocanate reductase. Non-limiting examples of such inhibitors are RNAi molecules, such as miRNA and siRNA.

In an embodiment, the inhibitor is bacterial histidine ammonia lyase inhibitor. The inhibitor could achieve its inhibiting effect by binding to and blocking or at least interfering with the enzymatic action of bacterial histidine ammonia lyase. Alternatively, the inhibitor could inhibit or at least reduce the transcription, translation and/or any post-translational processing of bacterial histidine ammonia lyase. Non-limiting examples of such inhibitors are RNAi molecules, such as miRNA and siRNA.

Inhibitors of the ImP-producing bacteria include probiotics that could deplete the substrate. Succinate-producing bacteria might be a potential probiotics [4] that deplete the substrate and thereby reduce the amount of imidazole propionate produced by the bacteria. Other probiotics that could be used include *Lactobacillus, Bifidobacterium, Enterococcus, Bacteroides, Prevotella* and *Clostridium.* Specific antibiotics that may target ImP-producing bacteria include antibiotics that target at least one *Lactobacillus, Adlercreutzia, Clostridium, Desulfatibacillum, Eggerthella, Listeria, Klebsiella, Mycobacterium, Shewanella, Streptococcus, Enterobacter, Enterococcus, Fusobacterium,* and *Aerococcus,* which mostly belongs to *Firmicutes* and *Proteobacteria.* Thus, antibiotics targeting at least one these classes of bacteria may useful to achieve a reduction of imidazole propionate. An example includes, but is not limited to, amoxicillin.

The method may comprise administering an effective amount of at least one urocanate reductase inhibitor, at least one histidine ammonia lyase inhibitor, at least one inhibitor of an imidazole propionate producing bacteria, at least one inhibitor of p38y MAPK, at least one inhibitor of p38δ MAPK, at least one inhibitor of mTORC1 and/or at least one inhibitor of alternative p38 MAPK activation pathway.

The inhibitor or agent of the embodiments may be administered according to various routes. For instance, the inhibitor or argent may be administered for delivery directly (locally) to gastrointestinal tract or systemically, using appropriate means of administration that are known to the skilled person.

In an embodiment, the inhibitor, e.g., the derivative of imidazole propionate, of the embodiments is a non-absorbable inhibitor in order to preferentially target bacterial enzymes in the gastrointestinal tract, e.g., bacterial histidine ammonia lyase and/or urocanate reductase.

In another embodiment, the inhibitor could be an absorbable inhibitor, in particular if the inhibitor is directed towards a target in the subject, such as for inhibitors of p38y MAPK or of p38δ MAPK,
In accordance with the embodiments, the inhibitor or agent may be administered orally, intravenously, intraperitoneally, subcutaneously, buccally, rectally, dermally, nasally, tracheally, bronchially, topically, by any other patenteral route or via inhalation, in the form of a pharmaceutical preparation comprising the active ingredient in a pharmaceutically acceptable dosage form.

In therapeutic treatment of mammals, and especially humans, the inhibitor or agent may be administered alone, but will generally be administered as a pharmaceutical formulation in admixture with a pharmaceutically acceptable adjuvant, diluent or carrier, which may be selected with due regard to the intended route of administration and standard pharmaceutical practice.

As used herein, effective amount means an amount sufficient at least to ameliorate or prevent a symptom of one of the aforementioned conditions, i.e., T2D or IGT. The effective amount for a particular patient may vary depending on such factors as the state of the condition being treated, the overall health of the patient, the method of administration, the severity of side-effects, and the like.

A particular embodiment relates to a method of treating a subject having or being at risk of developing T2D or IGT. The method comprises measuring an amount of imidazole propionate in a body sample from the subject. The method also comprises determining that the subject has or is at a risk of developing IGT if the concentration of imidazole propionate is equal to or greater than a threshold concentration for IGT but less than a threshold concentration for T2D and/or determining that the subject has or is at a risk of developing T2D if the concentration of imidazole propionate is equal to or greater than the threshold concentration for T2D.

In a particular embodiment, the threshold concentration of imidazole propionate for IGT is 8 nM, preferably 9 nM, and more preferably 10 nM, and the threshold concentration of imidazole propionate for T2D 10 nM, preferably 11 nM, and more preferably 12 nM.

In another particular embodiment, the threshold concentration of imidazole for T2D is selected within an interval of from 10.7 nM up to 13.8 nM.

The method further comprises administering to the subject determined to have or be at risk of developing T2D or IGT an effective amount of an inhibitor selected from the group consisting of an urocanate reductase inhibitor, a histidine ammonia lyase inhibitor, an inhibitor of an imidazole propionate producing bacteria, an inhibitor of p38γ MAPK, an inhibitor of p38δ MAPK, an inhibitor of mTORC1, an inhibitor of alternative p38 MAPK activation pathway, and any combination thereof, and/or or an effective amount of an imidazole propionate derivative, thereby treating the subject determined to have or be at risk of developing T2D or IGT.

Another particular embodiment relates to a method of treating a subject having or being at risk of developing T2D or IGT. The method comprises measuring an amount of imidazole propionate in a body sample from the subject.

In a particular embodiment, an amount of imidazole propionate of at least 8 nM, preferably 9 nM, and more preferably 10 nM, but less than 10 nM, preferably 11 nM, and more preferably 12 nM, diagnoses the subject as having or being at risk of developing IGT and an amount of imidazole propionate of at least 10 nM, preferably 11 nM, and more preferably 12 nM, diagnoses the subject as having or being at risk of developing T2D, thereby diagnosing a subject as having or being at risk of developing T2D or IGT.

In another particular embodiment, an amount of imidazole above a threshold concentration of imidazole propionate selected within an interval of from 10.7 nM up to 13.8 nM diagnoses the subject as having or being at risk of developing T2D.

The method also comprises administering to the subject diagnosed as having or be at risk of developing T2D or IGT an effective amount of an inhibitor selected from the group consisting of an urocanate reductase inhibitor, a histidine ammonia lyase inhibitor, an inhibitor of an imidazole propionate producing bacteria, an inhibitor of p38γ MAPK, an inhibitor of p38δ MAPK, an inhibitor of mTORC1, an inhibitor of alternative p38 MAPK activation pathway, and any combination thereof, and/or or an effective amount of an imidazole propionate derivative, thereby treating the subject diagnosed as having or be at risk of developing T2D or IGT.

A further particular embodiment relates to a method of treating a subject having T2D or at risk of developing T2D. The method comprises administering to a subject having an amount of imidazole propionate of at least 10 nM, preferably 11 nM, and more preferably 12 nM" such as above a threshold concentration of imidazole propionate selected within an interval of from 10.7 nM up to 13.8 nM, in a body sample from the subject an effective amount of an inhibitor selected from the group consisting of an urocanate reductase inhibitor, a histidine ammonia lyase inhibitor, an inhibitor of an imidazole propionate producing bacteria, an inhibitor of p38y MAPK, an p38δ MAPK an inhibitor of alternative p38 MAPK activation pathway, an inhibitor of mTORC1, and any combination thereof, and/or or an effective amount of an imidazole propionate derivative.

Yet another particular embodiment relates to a method of treating a subject having IGT or at risk of developing IGT. The method comprises administering to a subject having an amount of imidazole propionate of at least 8 nM, preferably 9 nM, and more preferably 10 nM, but less than 10 nM, preferably 11 nM, and more preferably 12 nM, such as above a threshold concentration of imidazole propionate selected within an interval of from 10.7 nM up to 13.8 nM, in a body sample from the subject an effective amount of an inhibitor selected from the group consisting of an urocanate reductase inhibitor, a histidine ammonia lyase inhibitor, an inhibitor of an imidazole propionate producing bacteria, an inhibitor of p38y MAPK, an inhibitor of p38δ MAPK, an inhibitor of alternative P38 MAPK activation pathway, an inhibitor of mTORC1, and any combination thereof, and/or or an effective amount of an imidazole propionate derivative.

The embodiments also relates to the above mentioned inhibitors for use in preventing, inhibiting or treating T2P or IGT in a subject. These embodiments thereby defines the following uses.

An urocanate reductase inhibitor for use in preventing, inhibiting or treating T2D or IGT in a subject.

A histidine ammonia lyase inhibitor for use in preventing, inhibiting or treating T2D or IGT in a subject.

An inhibitor of an imidazole propionate producing bacteria for use in preventing, inhibiting or treating T2D or IGT in a subject.

An inhibitor of p38y MAPK for use in preventing, inhibiting or treating T2D or IGT in a subject. In an embodiment, p38y MAPK inhibitor is selected from a group consisting of 1-(5-*tert*-butyl-2-*p*-tolyl-2H-pyrazol-3-yl)-3-[4-(2-morpholin-4-yl-ethoxy)naphthalen-1-yl]urea (BIRB796), pirfenidone and an RNAi molecule.

An inhibitor of p38δ MAPK for use in preventing, inhibiting or treating T2D or IGT in a subject. In an embodiment, p38y MAPK inhibitor is selected from a group consisting of 1-(5-*tert*-butyl-2-*p*-tolyl-2H-pyrazol-3-yl)-3-[4-(2-morpholin-4-yl-ethoxy)naphthalen-1-yl]urea (BIRB796) and an RNAi molecule.

An inhibitor of alternative p38 MAPK activation pathway for use in preventing, inhibiting or treating T2D or IGT in a subject.

An inhibitor of mTORC1 for use in preventing, inhibiting or treating T2D or IGT in a subject. In an embodiment, the mTORC1 inhibitor is selected from a group consisting of rapamycin, everolimus, AZD8055, temsirolimus, PP242 (torkinib), torin1, and WYE-125132.

An imidazole propionate derivative for use in preventing, inhibiting or treating T2D or IGT in a subject. In an embodiment, the imidazole propionate derivative is chloroimadazole propionate.

### EXAMPLES

Type 2 diabetes is associated with an altered gut microbiota, which may contribute to the development of this disease. The gut microbiota is known to modify the levels of amino acid-derived signaling molecules, but it is not clear if and how microbiota-dependent regulation of amino acid metabolism influences host metabolism. Herein, it is shown that the microbially upregulated histidine-derived metabolite imidazole propionate is present at higher concentrations in subjects with type 2 diabetes. Using fecal microbiota from individuals with and without type 2 diabetes, a dramatically increased production of imidazole propionate is demonstrated *in vitro* only in the presence of diabetes-associated microbiota. It is also shown that imidazole propionate reduces insulin receptor substrate protein levels and thereby impairs insulin signaling through alternative p38 mitogen-activated protein kinase-mediated activation of mTORC1. Together, these findings indicate that the microbial metabolite imidazole propionate contributes to the pathogenesis of type 2 diabetes.

### Imidazole propionate is associated with insulin resistance in humans

Bacterial metabolites produced in the gut reach the liver through the portal vein and then enter the systemic circulation. Therefore, to identify microbially upregulated amino acid-derived metabolites that may contribute to insulin resistance and type 2 diabetes, we first performed untargeted metabolomics on the portal blood of five obese subjects (body mass index (BMI) >40) with type 2 diabetes and ten BMI-matched subjects without type 2 diabetes, see Table 2. Only four amino acid-derived metabolites, dopamine sulfate, glutamate, imidazole propionate and N-acetylputrescine, were significantly higher, i.e. false discovery rate (FDR) <0.1, in the portal blood of subjects with type 2 diabetes, see Table 3.

**Table 2 - Characteristics of the obese cohort used to obtain portal blood for global metabolite analysis**

| | **Subjects without type 2 diabetes** | **Subjects with type 2 diabetes** | *P* |
|---|---|---|---|
| **n** | 10* | 5 | - |
| **Men, n (%)** | 2 (20.0) | 4 (80.0) | 0.025 |
| **Age, years** | 47.7 ± 3.2 | 52.6 ± 4.1 | 0.499 |
| **BMI, kg/m²** | 41.0 ± 1.2 | 42.6 ± 2.5 | 0.930 |
| **Fasting glucose, mM** | 5.6 ± 0.1 | 8.0 ± 0.4 | 0.002 |
| **Fasting insulin, µU/ml** | 10.5 ± 1.6 | 22.5 ± 7.5 | 0.059 |
| **HOMA-IR** | 2.7 ± 0.4 | 7.6 ± 2.1 | 0.004 |
| **HbA1c, mmol/mol** | 38.9 ± 0.9 | 54.0 ± 1.1 | 0.002 |

| | | | |
|---|---|---|---|
| Abbreviations: BMI, body mass index; HOMA IR, homeostatic model assessment of insulin resistance. * *n* = 9 for fasting insulin and HOMA-IR measurements. Data are presented as mean ± s.e.m or as proportions. P values were calculated using Wilcoxon Rank-Sum Test for continuous variables and chi square test for categorical variables. | | | |

**Table 3 - Amino acid-derived metabolites that were significantly higher (FDR <0.1) in portal blood from obese subjects with versus without type 2 diabetes**

| **Metabolite** | **Subjects without type 2 diabetes (*n* = 10)** | **Subjects with type 2 diabetes (*n*= 5)** | CAS |
|---|---|---|---|
| **Dopamine sulfate** | 0.72 ± 0.11 | 1.45 ± 0.20 | |
| **Glutamate** | 1.12 ± 0.09 | 2.07 ± 0.20 | 56-86-0 |
| **Imidazole propionate** | 0.85 ± 0.13 | 3.09 ± 0.95 | 1074-59-5 |
| **N-acetylputrescine** | 0.99 ± 0.06 | 1.40 ± 0.10 | 18233-70-0 |

| | | | |
|---|---|---|---|
| Data are presented as mean ± s.e.m of scaled intensity from the global metabolite analysis | | | |

To focus on microbial metabolites only, we then investigated levels of these four metabolites in the global metabolomics profile of blood from C57BL/6J germ-free (GF) and conventionally raised (CONVR) mice. We showed that imidazole propionate but not glutamate was present at higher levels both in the portal vein and vena cava blood from the CONVR mice, see Fig. 1a, dopamine sulfate and N-acetylputrescine were not detected. We next developed a targeted method for imaidazole propionate measurements and verified that the peak in the mice plasma had the same retention time and fragmentation pattern as those of the synthetic reference substance (CAS 1074-59-5, Santa Cruz). Targeted measurements of imidazole propionate in the portal and peripheral blood from the initial human cohort: (1) confirmed that concentrations of imidazole propionate were higher in the portal blood of subjects with type 2 diabetes and (2) showed that concentrations of imidazole propionate were also higher in the peripheral blood of subjects with type 2 diabetes compared with those without type 2 diabetes, see Fig. 1b.

To directly address if imidazole propionate is a microbially produced metabolite, we used an *in vitro* gut simulator, which mimics the anaerobic, reducing gut environment. After one week stabilization of the microbial communities from obese subjects with or without type 2 diabetes, we challenged the communities with 10 mM histidine and measured levels of imidazole propionate and its precursor urocanate, see Fig. 2, for 7 h. The bacterial community from the subject without type 2 diabetes rapidly produced urocanate to levels that reached a peak 3 h after histidine supplementation. Imidazole propionate was barely detected at any time point, see Fig. 1c, left. The type 2 diabetes-associated microbiota also produced urocanate immediately after histidine supplementation but at a lower rate. Notably, imidazole propionate production was observed 1.5 h after histidine supplementation, see Fig. 1c, right, reflecting the precursor and product relationship between urocanate and imidazole propionate. These observations suggest that type 2 diabetes-associated microbiota can shift the metabolism of urocanate from conventional *Hut* pathways to produce glutamate to the lesser known pathway to produce imidazole propionate, see Fig. 2.

To validate these findings, we performed batch culture and compared imidazole propionate production from histidine in bacterial communities from subjects with and without type 2 diabetes (n = 5 per group, again from the first cohort). End-point measurements showed that higher concentrations of imidazole propionate were produced in the presence of the type 2 diabetes-associated microbiota (Fig. 1e). These observations suggest that type 2 diabetes-associated microbiota shunt urocanate to a lesser known pathway to produce imidazole propionate.

The bacterial urocanate reductase (UrdA), which catalyzes the production of imidazole propionate from urocanate, has only recently been discovered (in *Shewanella oneidensis*). We performed large-scale screening for potential UrdA based on FAD active sites and identified 812 UrdA homologs in bacterial genomes: 639 with conserved "H" and 173 without "H" (replaced with "Y" or "M"). Next, we compared the ability of bacteria with "H" or without "H" to produce imidazole propionate from histidine or urocanate. We showed that bacteria with "H"-UrdA homologs did not produce imidazole propionate whereas bacteria with "Y" or "M"-UrdA homologs produced imidazole propionate (Fig. 1f), indicating that we could predict bacteria that produce imidazole propionate (i.e., "Y" or "M"-UrdA homologs are authentic urocanate reductases).

To validate our observation of increased concentrations of imidazole propionate in the peripheral blood of those with type 2 diabetes, we initially recruited 584 middle-age individuals from the Swedish population, and then in extended variant, 649 individuals, and divided them into four groups according to their level of glucose tolerance, see Tables 4A and 4B. We found significantly higher levels of imidazole propionate in those with impaired glucose tolerance (P = 0.020) or treatment-naive type 2 diabetes (P = 0.002) compared with those with normal glucose tolerance, see Fig. 1d, after correcting for BMI and sex. The imidazole propionate showed a tendency towards higher levels in those with impaired glucose tolerance (*P* = 0.057) and remained significantly higher in those with treatment-naive type 2 diabetes (*P* = 0.047) compared with those with normal glucose tolerance. We found significantly higher levels of imidazole propionate across the groups (*P*<0.0001) and those with treatment-naive type 2 diabetes had higher levels of imidazole propionate compared with those with normal glucose tolerance after correcting for the potential confounding factors BMI, sex and age (*P*<0.0001). By contrast, urocanate levels did not differ between the groups, see Fig. 1d.

**Table 4A- Clinical characteristics of 584 middle-age individuals from the Swedish population divided into four groups according to their level of glucose tolerance**

| | **NGT** | **IFG** | **IGT** | **T2D** | *P* |
|---|---|---|---|---|---|
| *n* | 303 | 119 | 142 | 20 | - |
| **Men, *n* (%)** | 116 (38.3) | 55 (46.2) | 71 (50.0) | 12 (60.0) | 0.039 |
| **Age, years** | 57.9 ± 0.3 | 57.8 ± 0.4 | 59.9 ± 0.3 | 58.7 ± 1.0 | <0.001 |
| **BMI, kg/m²** | 27.3 ± 0.2 | 27.4 ± 0.4 | 28.1 ± 0.4 | 31.6 ± 1.1 | 0.001 |
| **Fasting glucose, mM** | 5.2 ± 0.3 | 6.4 ± 0.2 | 5.6 ± 0.1 | 7.5 ± 0.5 | <0.001 |
| **Fasting insulin, µU/ml** | 5.4 ± 0.2 | 5.9 ± 0.3 | 7.1 ± 0.4 | 9.4 ± 1.2 | <0.001 |
| **HOMA-IR** | 1.2 ± 0.4 | 1.7 ± 0.9 | 1.8 ± 0.1 | 3.2 ± 0.4 | <0.001 |
| **HbA1c, mmol/mol** | 34.2 ± 0.2 | 36.0 ± 0.3 | 36.7 ± 0.3 | 48.6 ± 3.4 | <0.001 |

| | | | | | |
|---|---|---|---|---|---|
| Abbreviations: NGT, normal glucose tolerance; IFG, impaired fasting glucose; IGT, impaired glucose tolerance; T2D, type 2 diabetes; BMI, body mass index; HOMA-IR, homeostatic model assessment insulin resistance. Data are presented as mean ± s.e.m or as proportions. P values were calculated using Wilcoxon Rank-Sum Test for continuous variables and chi square test for categorical variables. | | | | | |

**Table 4B - Clinical characteristics of 649 middle-age individuals from the Swedish population divided into four groups according to their level of glucose tolerance**

| | **NGT** | **IFG** | **IGT** | **T2D** | *P* |
|---|---|---|---|---|---|
| ***n*** | 335 | 119 | 142 | 53 | - |
| **Men, *n* (%)** | 200 (59.7) | 64 (53.8) | 71 (50.0) | 26 (49.1) | 0.160 |
| **Age, years** | 57.9 ± 0.2 | 57.9 ± 0.4 | 59.9 ± 0.3 | 58.6 ± 0.6 | <0.001 |
| **BMI, kg/m²** | 27.2 ± 0.2 | 27.4 ± 0.4 | 28.1 ± 0.4 | 31.7 ± 0.8 | <0.001 |
| **Fasting glucose, mM** | 5.2 ± 0.0 | 6.4 ± 0.0 | 5.6 ± 0.1 | 7.6 ± 0.4 | <0.001 |
| **HbA1c, mmol/mol** | 34.2 ± 0.2 | 36.0 ± 0.3 | 36.7 ± 0.3 | 45.9 ± 1.6 | <0.001 |

| | | | | | |
|---|---|---|---|---|---|
| Abbreviations: NGT, normal glucose tolerance; IFG, impaired fasting glucose; IGT, impaired glucose tolerance; T2D, type 2 diabetes; BMI, body mass index. Data are presented as mean ± s.e.m or as proportions. P values were calculated using Wilcoxon Rank-Sum Test for continuous variables and chi square test for categorical variables. | | | | | |

### Imidazole propionate impairs glucose tolerance and insulin signaling

To directly test whether imidazole propionate could affect glucose metabolism, we injected imidazole propionate intraperitoneally into GF mice for 3 days. This treatment resulted in circulating concentrations of imidazole propionate of around 13 nM without affecting urocanate levels, see Fig. 3a. Importantly, these low concentrations of imidazole propionate were sufficient to induce significant glucose intolerance, see Fig. 3b, supporting a role for imidazole propionate as a plausible contributor to impaired glucose metabolism *in vivo.* We also observed that hepatic expression of *G6pase* and *Pepck*, genes for key rate-limiting gluconeogenic enzymes, were significantly increased in imidazole propionate-injected GF mice compared with controls, see Fig. 3c. However, imidazole propionate did not affect hepatic expression of *Srebp1c* and *Fasn,* genes for key lipogenic enzymes, see Fig. 3c.

We next investigated the effect of imidazole propionate injections on key proteins involved in insulin signaling. We found that imidazole propionate did not affect hepatic levels of the insulin receptor (IR) but reduced hepatic levels of insulin receptor substrate 1 and 2 (IRS1 and IRS2) in GF mice, see Fig. 3d. These reductions occurred at the posttranscriptional levels since there were no significant changes in hepatic *Irs1* and *Irs2* mRNA levels, see Fig. 4.

Next we investigated whether 3-day injections of imidazole propionate could affect glucose metabolism and insulin signaling in CONVR mice (housed in metabolic cages). We showed that this treatment increased circulating concentrations of imidazole propionate by around 4 fold without affecting urocanate levels (Fig. 3f). Of note, imidazole propionate treatment impaired glucose tolerance (Fig. 3g) without any significant effect on body weight, liver and WAT weight or food intake in CONVR mice. Energy expenditure in the early dark phase was significantly reduced by imidazole propionate in parallel with reductions in locomotor activity; however, total locomotor activity was not significantly reduced by imidazole propionate treatment.

In contrast to our observations in GF mice, imidazole propionate treatment did not reduce IRS levels in liver, muscle or WAT in CONVR mice. We therefore further investigated the role of imidazole propionate on insulin signaling using primary hepatocytes. Although treatment with imidazole propionate for 20 h reduced IRS in hepatocytes (Fig. 3h), consistent with the response to imidazole propionate in GF liver, 8 h treatment with imidazole propionate did not affect IRS (Fig. 3e). However, this short term treatment blunted insulin-induced IRS1 Y612 phosphorylation (Fig. 3e), which is required for insulin-stimulated activation of IRS-1-associated PI3K and downstream Akt. Akt is fully activated when both residues S473 and T308 are phosphorylated21, but Akt S473 phosphorylation and T308 phosphorylation have been reported to mediate different physiological roles. Here we showed that 8 h treatment with imidazole propionate reduced insulin-induced Akt S473 - but not Akt T308 - phosphorylation (Fig. 3e). Specific removal of insulin-stimulated Akt S473 phosphorylation but not T308 phosphorylation in the liver or fat is sufficient to impair glucose tolerance, suggesting the importance of Akt S473 phosphorylation in mediating the beneficial response of insulin.

Of note, 8 h treatment of primary hepatocytes with imidazole propionate induced basal Akt phosphorylation at both S473 and T308, thereby reducing the responsiveness of Akt to insulin (Fig. 3e). We also noted that basal Akt S473 phosphorylation was higher in liver, muscle and WAT from 3-day imidazole propionate-injected versus vehicle-treated CONVR mice. Basal Akt activation is often observed in Western diet-induced insulin-resistant animals and diabetic leptin-deficient ob/ob mice, inhibits insulin-stimulated glucose uptake and can reduce insulin sensitivity by inhibiting insulin-stimulated IRS1 tyrosine phosphorylation and IRS1-associated PI3K activity.

By contrast with IRS protein levels, there was no reduction in Akt phosphorylation, either S473 or T308, in liver from GF mice after treatment with imidazole propionate, see Fig. 3d. Of note, earlier studies have shown that feeding mice a high-fat diet for 8-12 weeks resulted in impaired glucose tolerance together with reduced levels of IRS1 proteins but no change or an increase in Akt phosphorylation. Similarly, in primary hepatocytes incubated with imidazole propionate for 14 h, we observed a dramatic reduction in IRS1 combined with an increase in basal Akt phosphorylation at S473 and T308, see Fig. 3e. Despite the increased basal Akt activation, insulin-induced Akt S473 phosphorylation was lower in primary hepatocytes exposed to imidazole propionate, see Fig. 3e. By contrast, insulin-induced Akt T308 phosphorylation was higher in primary hepatocytes exposed to imidazole propionate compared with vehicle-treated hepatocytes, see Fig. 3e. Previous studies have shown that specific removal of insulin-induced Akt S473 phosphorylation but not T308 phosphorylation in the liver or fat is sufficient to impair glucose tolerance, suggesting the importance of Akt S473 phosphorylation in mediating the beneficial response of insulin.

Together, these data show that imidazole propionate worsens glucose tolerance, which could be at least partially explained by reduced IRS protein levels and reduced insulin-induced Akt S473 phosphorylation.

Our results thus show that imidazole propionate has negative impacts on insulin signaling at the level of IRS, potentially by increasing basal Akt activation and thereby reducing insulin stimulated tyrosine phosphorylation.

### Imidazole propionate modulates the amino acid sensing pathway

Potential pathways that could link imidazole propionate to IRS reduction include those involving c-Jun N-terminal kinase (JNK) or mechanistic target of rapamycin complex 1 (mTORC1). We first showed that the JNK inhibitors SP600125 or BI78D3 did not restore imidazole propionate-induced IRS1 reduction in primary hepatocytes, see Fig. 5, excluding a role for the JNK pathway. By contrast, we showed that the mTORC1 inhibitor rapamycin and torin 1 inhibited the imidazole propionate-induced reduction of IRS in these cells, see Figs. 6a and 6i. Furthermore, S6K1 phosphorylation, which is a marker of mTORC1 activation, was increased in primary hepatocytes incubated with imidazole propionate, and this response was reversed by rapamycin and torin 1, see Figs. 6a and 6i. These results suggest that imidazole propionate reduces IRS protein levels by affecting signaling molecules upstream of mTORC1. We also showed that 3-day treatment of GF mice with imidazole propionate increased S6K1 phosphorylation without affecting mTORC1 S2481 autophosphorylation in the liver, see Fig. 6b. Because mTOR S2481 autophosphorylation is known to be increased by insulin but not by amino acids when measured in total cell lysates, our results suggest activation of the amino acid sensing pathway by imidazole propionate.

To further investigate the role of imidazole propionate in the amino acid sensing pathway, we incubated amino acid-deprived primary hepatocytes with imidazole propionate. In the absence of amino acids, incubation with imidazole propionate for 2 h strongly induced S6K1 phosphorylation, see Fig. 6c. We also showed that incubation with imidazole propionate for 1 h in the human cell line HEK293 cells induced S6K1 phosphorylation at levels comparable to those observed in the presence of amino acids, see Fig. 6d.

Although our data suggested that imidazole propionate shares the signaling machinery of amino acid-induced mTORC1 activation, imidazole propionate is not a typical amino acid that can be utilized by cells. To clarify differences or similarities between imidazole propionate and amino acids in mTORC1 activation, we incubated amino acid-deprived primary hepatocytes with imidazole propionate. In the absence of amino acids, incubation with imidazole propionate for 2 h strongly induced S6K1 phosphorylation (Fig. 6c). We also showed that incubation with imidazole propionate for 1 h in the human cell line HEK293 cells induced S6K1 phosphorylation at levels comparable to those observed in the presence of amino acids. Leucine, which is known to potently activate mTORC1, increased S6K1 phosphorylation in HEK293 cells after only 15 min, but its effect was transient and not as strong as the response to imidazole propionate observed at later time points. However, imidazole propionate induced S6K1 phosphorylation in HEK293 after only 30 min in the presence of amino acids. These results suggest differences between typical amino acids and imidazole propionate in the activation of mTORC1.

By performing immunoprecipitation experiments, we showed that 2 h incubation with imidazole propionate in HEK293 cells specifically modulated mTORC1 and not mTORC2. Of note, although both imidazole propionate and amino acids phosphorylated mTORC1 to a similar extent, only imidazole propionate induced Akt phosphorylation. In agreement with earlier observations, we showed that insulin-induced Akt activation did not activate mTORC1 in the absence of amino acids, in contrast to imidazole propionate-induced Akt activation. We also showed that mTORC1 and Akt were independently activated by imidazole propionate in the absence of amino acids, but that Akt could act as an upstream regulator of mTORC1 in the presence of amino acids. Together, these results indicate that imidazole propionate activates mTORC1 in a different manner than either amino acids or insulin.

IRS1 protein levels were not reduced by imidazole propionate after short incubation times, up to 2 h, in amino acid-deprived cells, see Figs. 6c and 6d. Because previous studies have shown that IRS1 degradation is preceded by phosphorylation of IRS1 at S636 and S639, which is induced by mTOR/S6K1 hyperactivation and subsequent ubiquitin/proteasome-mediated degradation, we next investigated if imidazole propionate could induce IRS1 S636/S639 phosphorylation in amino acid-deprived HEK293 cells. We showed that incubation with imidazole propionate for 2 h induced IRS1 S636/S639 phosphorylation without affecting IRS1 protein levels. 8 h of imidazole propionate dramatically reduced IRS1 levels and resulted in a mobility shift, see Fig. 6e, which is known to be caused by IRS1 serine phosphorylation. These results suggest that imidazole propionate promotes IRS1 degradation in response to IRS1 serine phosphorylation.

Insulin activates mTORC1 through activation of Akt, which relieves inhibitory effects of TSC on Rheb-GTP, a potent activator of mTORC1. In contrast, persistent activation of mTORC1 inhibits insulin-induced Akt activation through a negative-feedback loop towards IRS. However, insulin cannot activate mTORC1 in the absence of amino acids, and we showed that insulin-induced Akt activation did not lead to mTORC1 activation, as measured by S6K1 phosphorylation, in the absence of amino acids or imidazole propionate, see Fig. 6e. Furthermore, pretreatment with imidazole propionate for 8 h resulted in mTORC1 activation and reduction of IRS1 together with increased basal activation of Akt and reduced responsiveness of Akt S473 to insulin, see Fig. 6e, further supporting the concept that mTORC1-mediated inhibition of insulin signaling occurs via IRS1 degradation.

Incubation with imidazole propionate for 8 h promoted a further mobility shift of IRS1 and dramatically reduced IRS1 levels (Fig. 6e). We also showed that 6 h treatment with imidazole propionate promoted ubiquitination of IRS1. Together, these results suggest that imidazole propionate promotes IRS degradation through an mTORC1-dependent pathway and support the concept that IRS degradation is preceded by IRS S636/S639 phosphorylation and ubiquitination.

### Imidazole propionate promotes lysosomal localization of mTORC1

Earlier studies have shown that mTORC1 activation by amino acids requires lysosomal localization of mTORC1, which is mediated by the formation of a complex between the adaptor protein p62, the active Rag heterodimer RagB-GTP/RagC-GDP, Raptor and mTORC1. Here we showed that imidazole propionate induced lysosomal localization of mTOR, see Fig. 6f. We, thus, next investigated whether imidazole propionate could facilitate the formation of the active complex. By performing immunoprecipitation with FLAG-tagged mTOR and monitoring interaction with HA-tagged Raptor, Myc-tagged Rag GTPases and p62, we showed that imidazole propionate induced formation of the mTORC1-Rag heterodimer complex-p62 to levels comparable to those achieved in cells expressing the constitutively active RagB-GTP/RagC-GDP, see Fig. 6g. Next, we determined the involvement of Rag GTPases in imidazole propionate-induced S6K1 activation. We showed that RagA/B-GDP and RagC/D-GTP, which are inactive Rag GTPases, blocked the imidazole propionate-induced S6K1 phosphorylation, see Fig. 6h. Moreover, the active Rag GTPases RagA/B-GTP did not further increase S6K1 phosphorylation, see Fig. 6h, suggesting that imidazole propionate activates mTOR through the activation of Rag GTPases.

### Imidazole propionate but not other histidine derivatives induces p62 phosphorylation

Phosphorylation of the adaptor protein p62 at T269 and S272 has recently been shown to be critical for amino acid induction of mTORC1 activation at the lysosome. Here we showed that p62 phosphorylation was induced in primary hepatocytes by incubation with imidazole propionate, see Fig. 7a. Furthermore, this response was not affected by the mTORC1 inhibitor rapamycin, see Fig. 7a, indicating that mTORC1 is downstream of p62 phosphorylation.

Imidazole propionate promoted a concentration- and time-dependent increase in p62 phosphorylation in HEK293 cells, with activation observed at concentrations as low as 10 nM in the absence of amino acids, see Fig. 7b. S6K1 and IRS1 S636/S639 phosphorylation were not as sensitive to imidazole propionate as p62, see Fig. 7b, supporting a role for p62 phosphorylation upstream of mTORC1 activation. Of note, neither histidine nor its degradation products, i.e., urocanate and glutamate, nor the structurally similar bacterial metabolite indole propionate phosphorylated p62 or S6K1, see Fig. 7c, highlighting the specificity of imidazole propionate.

### Alternative p38 MAPK promotes imidazole propionate-induced signaling

A recent study reported that the p38 mitogen-activated protein kinase (MAPK) p38δ is required to mediate the amino acid-induced activation of p62/mTORC1, and we therefore investigated whether p38δ is also involved in the imidazole propionate-induced activation of p62/mTORC1. p38δ is one of four p38 MAPK isoforms that exist in mammalian cells, and these isoforms can be divided into two subsets: p38α/p38β and p38γ/p38δ, also denoted alternative p38MAPK. p38α/p38β but not p38γ/p38δ is inhibited by the pyridinyl imidazole inhibitor SB20219037 whereas BIRB796 inhibits the entire p38 subfamily. Here, we showed that BIRB796 but not SB202190 inhibited the imidazole propionate-induced phosphorylation of p62 and S6K1, see Fig. 7d, indicating that p38γ/p38δ but not p38α/p38β is a downstream target of imidazole propionate.

To determine whether p38δ or p38γ is involved in imidazole propionate-induced p62/mTORC1 activation, we used siRNA to knock-down the two isoforms separately. Knock-down of p38δ only had a small inhibitory effect on phosphorylation of p62 and S6K1 and did not affect IRS1 phosphorylation induced by incubation with imidazole propionate for 1 h, see Fig. 7e and Fig. 8a. In contrast, p38y knock-down almost completely blocked imidazole propionate-induced phosphorylation of p62, S6K1, IRS1 S636/S639 and Akt, see Fig. 7e and Fig. 8b. An earlier study reported that p38γ/p38δ promotes mTOR activation by phosphorylating and, thus, promoting the degradation of the mTORC1 and mTORC2 inhibitor DEP domain-containing mTOR-interacting protein (DEPTOR). However, we showed that DEPTOR level was not affected by imidazole propionate in cells transfected with either control or p38δ/p38γ siRNA, see Fig. 7e.

Since, p38y knockdown prevented imidazole propionate-induced signaling, we then asked whether imidazole propionate could directly activate the kinase activity of p38y. By performing an *in vitro* kinase assay, we showed that p62 phosphorylation, at T269 and S272, was induced by p38y in the presence of 50 µM ATP, which was further increased with 200 µM ATP, see Fig. 7f. Importantly, imidazole propionate enhanced p62 phosphorylation in a concentration-dependent manner, see Fig. 7f, indicating that imidazole propionate could directly activate p38y.

The activation of p38MAPK including p38y is known to be mediated by phosphorylation on T180/Y182 by upstream kinases. ATP itself did not induce p38y autophosphorylation; however, to our surprise, imidazole propionate together with ATP induced p38y autophosphorylation. Although upstream kinase-mediated p38 phosphorylation is the conventional mechanism to activate p38, there have been studies supporting that p38 can autophosphorylate and thus activate itself.

### Akt S473 and Akt T308

Akt kinase can be fully activated when both residues S473 and T308 are phosphorylated. However, specific removal of insulin-stimulated Akt S473 phosphorylation but not T308 phosphorylation in the liver or fat is sufficient to impair glucose tolerance, suggesting the importance of Akt S473 phosphorylation in mediating beneficial response of insulin. In contrast, Akt T308 phosphorylation but not S473 phosphorylation is correlated with survival of acute myeloid leukemia patients and Akt kinase activity in human non-small cell lung cancer, possibly suggesting that Akt T308 in mediating cell growth and survival. Imidazole propionate increased basal Akt phosphorylation at S473 and T308 in primary hepatocytes (Fig. 3e). However, insulin-induced Akt phosphorylation at S473 was lower in primary hepatocytes exposed to imidazole propionate (Fig. 3e), possibly due to reduced IRS1 tyrosine phosphorylation.

### Comparison with Leucine

Leucine is one of the potent activator of mTORC1 in amino acid sensing pathway. Leucine-induced mTORC1 activation was rapid (within 15 min) but transient in the absence of amino acids. In contrast, imidazole propionate-induced mTORC1 activation was not observed until 30 min of incubation but was stronger after 2 h of treatment. In the absence of amino acids, mTORC1 is localized at the cytoplasm and not at the lysosome where all the machinery for amino acid-induced mTORC1 activation locates6. In the presence of amino acids, imidazole propionate also induced S6K1 phosphorylation within 30 min of incubation possibly due to pre-localized mTORC1 at the lysosome, suggesting that imidazole propionate might not be as efficient as amino acids in recruiting mTORC1 to the lysosome.

### Specificity of imidazole propionate on mTORC1 and unique character of imidazole propionate in the regulation of Akt

Since imidazole propionate activates amino acid sensing pathway, we further investigated its impact on mTOR modification. mTOR, S2448 and S2481 phosphorylation of mTOR are known to be associated with mTORC1 and mTORC2, respectively7. mTOR S2448 phosphorylation in mTORC1 (isolated by Raptor immunoprecipitation) was strongly induced by imidazole propionate or amino acids but not for mTOR S2481 phosphorylation in mTORC1. However, mTOR S2448 or S2481 phosphorylation in mTORC2 (isolated by Rictor immunoprecipitation) was unaffected by imidazole propionate; also, *in vitro* mTORC2 kinase activity towards Akt was not altered by imidazole propionate. These data suggest that imidazole propionate has more specificity towards mTORC1 pathway than mTORC2 pathway.

Imidazole propionate was different from amino acids since amino acids cannot induce Akt phosphorylation. Also, imidazole propionate was different from insulin because insulin cannot activate mTORC1 in the absence of amino acids. Akt is upstream activator for mTORC1 in the presence of amino acids; thus, we determined whether imidazole propionate-induced basal activation of Akt is responsible for mTORC1 activation. A highly selective allosteric inhibitor of Akt, MK2206 efficiently blocked the imidazole propionate-induced Akt activation but which did not affect imidazole propionate-induced S6K1 phosphorylation, suggesting that mTORC1 and Akt can be independently activated by imidazole propionate. In the presence of amino acids, however, Akt can act as an upstream regulator of mTORC1 shown by MK2206 mediated inhibition in imidazole propionate-induced S6K1 phosphorylation. These suggest that imidazole propionate has unique property in its signal transduction.

### Detailed action of imidazole propionate on IRS

Although mTORC1-mediated IRS serine phosphorylation was well studied, it was not clear whether mTORC1 itself or mTORC1-activated S6K1 is responsible for this event. Thus, we depleted mTORC1 or S6K1 through Raptor siRNA or S6K1 siRNA, respectively. These showed that mTORC1 is responsible for imidazole propionate-induced modification of IRS since Raptor silencing caused inhibition of IRS S636/S639 phosphorylation and partial blockade of IRS mobility shift. Although we showed imidazole propionate caused IRS S636/S639 phosphorylation and ubiquitination, it needs to be further investigated whether this S636/S639 phosphorylation is responsible for ubiquitination. Also, it is still possible that imidazole propionate can induce other modification of IRS.

In this study, we identified imidazole propionate as a microbially upregulated histidine-derived metabolite that is increased in patients with type 2 diabetes. We also showed that imidazole propionate treatment impairs glucose tolerance in mice and leads to reduced IRS protein levels. In addition, we investigated potential signaling pathways and provide evidence to show that imidazole propionate: (1) activates alternative p38 MAPK/mTORC1, which phosphorylates IRS proteins and thereby targets them for degradation; and (2) promotes basal Akt activation through p38y, which may further reduce the responsiveness to insulin, see Fig. 7g.

Our observation that imidazole propionate is present at similar concentrations in the portal and peripheral blood indicates that this metabolite is not metabolized and could thus have direct systemic effects. Hyperinsulinemia and BCAAs have been suggested to contribute to insulin resistance by affecting basal Akt and mTORC1 activation, respectively; however, insulin levels decline in the late stage of type 2 diabetes and it is still unclear whether BCAAs are a causative factor for insulin resistance and type 2 diabetes (toxic BCAA metabolites rather than BCAAs themselves seem to contribute to the metabolic syndrome). The imidazole propionate signaling pathway identified in our study helps to explain how activation of basal Akt and mTORC1 might contribute to the development of the metabolic syndrome. Furthermore, we identified alternative p38 MAPK, which has a broad spectrum of tissue expression as a target of imidazole propionate. Both p38y and p38δ are implicated in pathological conditions including impaired glucose tolerance. Since imidazole propionate altered p38 kinase activity *in vitro,* it will potentially affect other related kinases and activate downstream signaling pathways in addition to mTORC1.

Histidine degradation pathways leading to the production of urocanate and glutamate are well conserved both in bacteria and humans. However, the bacterial urocanate reductase (*urdA*), which catalyzes the production of imidazole propionate from urocanate, has only recently been identified. We did not identify any *urdA* homologs with >30 % sequence identity in the human genome, further supporting the concept that imidazole propionate is microbially produced. We predicted 812 UrdA homologs in bacterial genomes, and showed that bacteria with "Y" or "M" rather than the conserved "H" in the FAD active site could produce imidazole propionate. Among bacteria with "Y" or "M"- UrdA homologs we tested, *Streptococcus mutans* and *Eggerthella lenta* have been reported to be enriched in subjects with type 2 diabetes compared with healthy controls. Of note, we identified *Lactobacillus paraplantarum* as imidazole propionate-producing bacteria, which will have potential clinical importance considering the controversial roles of *Lactobacillus* on obesity and increased abundance in type 2 diabetes.

Although dietary fiber-derived microbial metabolites, such as butyrate and propionate, are produced in large quantities in the gut. They are only present at low concentrations in peripheral blood and, thus, they likely mediate effects on host metabolism indirectly through the hormonal and nervous systems in the gut. By contrast, circulating levels of the choline-derived microbial metabolites TMA and TMAO have been shown to predict cardiovascular disease in humans, suggesting that they have detrimental effects by a direct influence systemically. However, although trace amine-associated receptor 5 has been suggested as a target of TMA, its expression is restricted to the olfactory sensory neurons, and thus the direct target of TMA/TMAO remains unclear. Our observation that imidazole propionate is present at similar levels in the portal and peripheral blood indicates that this metabolite could also have direct systemic effects. Furthermore, we identified alternative p38 MAPK, which has a broad spectrum of tissue expression, as a target of imidazole propionate. Both p38y and p38δ are implicated in pathological conditions including impaired glucose tolerance, and thus identification of the signaling pathways induced by imidazole propionate as shown herein could form the basis to reveal novel drug targets to tackle insulin resistance and type 2 diabetes. Furthermore, following the recently described strategy to treat atherosclerosis by non-lethal inhibition of the microbial TMA lyase, our findings suggest that targeting specific imidazole propionate-producing bacteria or the bacterial enzymes producing imidazole propionate are a potential therapeutic strategies for type 2 diabetes.

### METHODS

### Human study populations

Two human cohorts were used in this study. The first cohort comprised five obese subjects with type 2 diabetes and ten BMI-matched subjects without type 2 diabetes who were undergoing laparoscopic Roux-and-Y gastric bypass surgery at the Slotervaart Hospital, Amsterdam, the Netherlands, see Table 2. Subjects were fasted overnight before the morning of the surgery, and a peripheral venous sample was taken just before the patient went into the operating room. Anthropometric measurements and a stool sample were also taken before surgery. During the surgery, a portal vein sample (vena puncture) was taken as previously described [1]. Fasting glucose (by glucose meters, Roche Diagnostics), insulin (by an electrochemiluminescence immunoassay, Roche Modular System) and HbA1c (Menarini Diagnostic) were measured in the peripheral blood. All participants gave informed consent before the surgery and the protocol was approved by the Medical Ethical Committee of the Slotervaart Hospital, Amsterdam, the Netherlands.

The second cohort initially comprised 584 subjects, and was then extended into 649 subject, from a randomly selected population sample of adults aged 50 to 64 years and living in the Gothenburg area, Sweden. These subjects were recruited from the census register. Exclusion criteria were: known diabetes; inflammatory diseases, such as Crohn's disease, ulcerative colitis, rheumatic diseases; treatment with steroids or immunomodulatory drugs; cancer (unless relapse free for the preceding 5 years); cognitive dysfunction. A 75 g oral glucose tolerance test (OGTT) was performed in the morning after an overnight fast, and fasting- and 2-h post load capillary blood glucose were measured. Subjects fulfilling criteria for type 2 diabetes were re-examined with a repeated OGTT within 3 weeks. Subjects were included in the study if they were found to have impaired fasting glucose (n = 119), impaired glucose tolerance (n = 142) or type 2 diabetes (n = 20/n = 53). In addition, approximately every fifth subject with normal glucose tolerance was included (n = 303/n = 335). Blood samples were obtained from all the participants. Examinations took place at the Wallenberg Laboratory, University of Gothenburg, Sweden. All participants gave informed consent and the study was approved by the Ethics Committee at Gothenburg University.

### Mice

15-week-old male C57BL/6J GF or CONVR mice were used for all the animal experiments. GF mice were maintained in flexible film isolators under a strict 12 h light cycle. GF status was verified regularly by PCR for bacterial 16S rDNA using the primers 27F (5'-GTTTGATCCTGGCTCAG-3', SEQ ID NO: 19) and 1492R (5'-CGGCTACCTTGTTACGAC-3', SEQ ID NO: 20). PCR reactions were performed with a 5-min preincubation at 95°C followed by 30 cycles of 30 sec at 94°C, 45 sec at 52°C and 90 sec at 72°C and then kept at 72°C for 7 min. All mice were fed an autoclaved chow diet (Labdiet) ad libitum. To measure microbial metabolites, blood was taken from the portal vein and vena cava of GF and CONVR mice after a 4 h fast.

To test the effect of imidazole propionate on glucose metabolism, 500 µg imidazole propionate in 200 µl water containing 1 % DMSO or vehicle (1 % DMSO in water) was injected intraperitoneally into GF mice twice per day for 3 days. For CONVR mice, 100 µg imidazole propionate was used since CONVR mice are continuously exposed to microbial metabolites and have longer retention time of organic solutes compared with GF mice. An intraperitoneal glucose tolerance test (using 1 g glucose per kg body weight) was then performed after a 4 h fast. Blood glucose levels were measured from tail blood using HemoCue201 + analyzer (HemoCue, Ängelholm, Sweden). Liver and plasma samples were collected after a 4 h fast. Mice were under isoflurane anesthesia and killed by cervical dislocation. Fresh liver was taken and snap-frozen in liquid nitrogen and stored at -80°C until protein extraction.

To assess the metabolic impact of imidazole propionate on CONVR mice, food intake, oxygen consumption and locomotor activity were measured using the PhenoMaster system (TSE systems, Bad Homburg, Germany) according to the manufacture's recommendation. Mice were singly housed for two days before being transferred to the PhenoMaster cages, and allowed an additional 2 days to acclimatize to the new cage before taking measurements. Parameters were automatically measured every 15 min for 5 days. During the measurements, the mice had free access to water and food. Mice were randomly divided into two weight matched groups and injected intraperitoneally either with imidazole propionate (100 µg) or vehicle twice a day (8 AM and 5 PM) for 3 days. Oxygen consumption (VO2) was calculated from measurements of oxygen and carbon dioxide concentration in the reference cage compared to the sample cage. Measurements of oxygen consumption and energy expenditure were adjusted to lean body mass, which was determined by MRI (EchoMRI, Houston, TX, USA).

All mice experiments were performed in our animal facility and were approved by the Ethics Committee on Animal Care and Use in Gothenburg, Sweden.

### Metabolite analysis

Global metabolite profiling analysis on (1) portal blood from obese humans with and without type 2 diabetes and (2) blood from the portal vein and vena cava of C57BL/6J GF and CONVR mice was performed by Metabolon (Durham, NC) using ultra high performance liquid chromatography and mass spectrometry (UPLC-MS/MS). In brief, samples were prepared using the automated MicroLab STAR® system (Hamilton Company). A recovery standard was added before the first step in the extraction process for quality control. Proteins were precipitated with methanol under vigorous shaking for 2 min (Glen Mills GenoGrinder 2000) followed by centrifugation. The resulting extract was divided into five fractions: one for analysis by UPLC-MS/MS with positive ion mode electrospray ionization, one for analysis by UPLC-MS/MS with negative ion mode electrospray ionization, one for LC polar platform, one for analysis by GC-MS, and one sample was reserved for backup. Samples were placed briefly on a TurboVap® (Zymark) to remove the organic solvent. For LC, the samples were stored overnight under nitrogen before preparation for analysis. For GC, each sample was dried under vacuum overnight before preparation for analysis.

For targeted measurement of imidazole propionate and urocanate, plasma samples were extracted with 3 volumes of ice-cold acetonitrile in 1.5 ml polypropylene tube and dried under a nitrogen flow. The samples were then reconstituted with 5 % HCI in n-butanol at 70°C for 1 h, allowing n-butyl ester to be formed. After derivatization, the samples were evaporated and reconstituted in 100 µl of water/acetonitrile (90:10) containing 50 nM of internal standards (labelled imidazole propionate and urocanate made from derivatization using d₁₀ labelled butanol). Samples (5 µl) were injected onto a C18 BEH column (2.1x100 mm with 1.7 µm particles; Waters, Milford, MA) using a gradient consisting of water with 0.1 % formic acid (A-phase) and acetonitrile with 0.1 % formic acid (B-phase). Mass spectrometric analysis was performed using an Infinity 1290 quaternary UPLC pump (Agilent, Santa Clara, CA) coupled to a QTRAP 5500 (ABSciex, Concord, Canada). The imidazole propionate and urocanate were detected by multiple reaction monitoring using the transitions 197/81 and 195/93, respectively. For the internal standards, the transitions 206/81 and 204/93 were used. Calibration curves of imidazole propionate and urocanate were made in water and treated the same way as the samples.

### In vitro gut simulator

A simulated human intestinal redox model (SHIRM) was used to directly investigate whether imidazole propionate could be a microbially produced metabolite. SHIRM is a two-chamber fermenter with an anaerobic luminal chamber and an oxygen feeder, which are separated by a CMI-7000S membrane (Membrane International, NJ) and continuously purged with nitrogen and oxygen, respectively. The oxygen chamber contained 100 mM potassium phosphate buffer. The feed for the luminal chamber consisted of (in g/l) arabinogalactan (1.0), pectin (2.0), xylose (1.5), starch (4.0), glucose (0.4), yeast extract (3.0), peptone (3.0), mucin type II (4.0), and cysteine (0.5). To simulate digestion processes, the feed was acidified to a pH of around 2 with 6 M HCI and neutralized with simulated pancreatic juice [(in g/l) NaHCO₃ (12.5), Ox gall bile salts (6.0) and pancreatin (0.9)] to pH 6.9. The luminal chamber was continuously fed with SHIRM feed (70:30 mix of the feed and pancreatic juice described above) at a rate giving a retention time of around 24 h and pH was maintained at 6.6-6.9 using a pH controller and dosing pump (Black Stone BL7912, Hanna Instruments, UK).

The SHRIM system was inoculated with an aliquot of the feces from one obese human with type 2 diabetes and one without type 2 diabetes (from the first cohort). A pre-culture was prepared anaerobically in a Coy chamber (5 % hydrogen, 10 % carbon dioxide and 85 % nitrogen) by adding 2 % fecal material to 5 ml of brain-heart infusion medium containing (in g/l) yeast extract (5), cellobiose (1), maltose (1), cysteine (0.5) and hemin (0.01). The pre-culture was incubated for 3 h at 37°C, and 2 % of the pre-culture was seeded into the luminal compartment of the SHRIM. The luminal communities were stabilized for 1 week before histidine challenge. Samples were collected for up to 7 h and imidazole propionate and urocanate levels were measured as described above.

### Batch culture of fecal samples

Fecal samples (100 mg) from donors with and without type 2 diabetes (from the first cohort) were inoculated in 5 ml BHI medium (as described above) and incubated overnight under strict anaerobic conditions in a Coy chamber at 37°C. After incubation, 1% (v/v) of the respective BHI precultures were inoculated in SHIRM feed (described above) supplemented with 10 mM histidine. After 36 h of incubation, supernatants were used for imidazole propionate and urocanate quantification. The levels of consumed urocanate were used to adjust those of produced imidazole propionate and log transformed fold-change over 0 h was calculated using these adjusted values.

### Computational prediction and experimental validation of UrdA-containing bacteria

UrdA was previously predicted to encode fumarate reductase because of its high sequence homology to the FAD- and FMN-binding domains of fumarate reductase, succinate dehydrogenase and L-aspartate oxidase. However, structural modelling of the FAD-binding domain from UrdA identified differences in amino acid sequences from those in fumarate reductase, which potentially gives substrate selectivity to urocanate. To screen for potential imidazole propionate-producing bacteria, we focused on amino acid sequences that are conserved in fumarate reductase from *Shewanella oneidensis* but replaced in potential UrdA. Since conserved "H"s in the active sites of enzymes are important for catalysis in various enzymes4-6, we focused on tyrosine 373 (Y373) in the FAD active sites of UrdA (Y373 corresponds to histidine (H) in fumarate reductase). Extensive protein screening followed by accurate domain alignment identified the existence of 812 UrdA homologs in 670 bacterial genomes. Of those, 639 were identified to have an "H" residue in their active sites whereas 173 have non-"H" residues such as "Y" or "M".

27 seed protein sequences (including 22 from KEGG annotations7) with high identity to UrdA from S. *oneidensis MR-1* were used to screen potential UrdA homologs among 16,403,888 proteins extracted from 4893 bacterial genomes. These data were downloaded from the NCBI ftp site ftp://ftp.ncbi.nlm.nih.gov/genomes/all/ on June 26, 2016. 658 protein hits were identified using BLASTP program (with the following settings: E value = 1e-4; identity=30%; coverage=70%) and those hits were further used as seeds to perform both the second round of protein BLAST and HMM profiling (with at least 50% coverage and score larger than 200), resulting in 2230 and 2526 protein hits, respectively. A further Pfam domain scan10 on all identified UrdA candidates revealed that 1774 sequences possess FAD domains. We thus extracted the FAD active sites of UrdA from S. *oneidensis MR-1* (extended to 30 amino acids upstream and downstream of Y373) and performed a third round of protein BLAST against the 1774 FAD-containing hits using the extracted Y373-containing domain as the query sequence. 812 protein hits were identified as the final UrdA homologs. Additional domain alignment was performed using ClustalX to annotate the corresponding active sites.

8 representative UrdA-containing bacterial strains (without conserved "H") were further subjected to experimental validation in pure cultures together with *Escherichia coli k12* and *Bifidobacterium longum NCC2705* as control (with conserved "H"). The maximum likelihood tree was based on extracted FAD domains from all 10 bacteria using MEGA (version 7) with default settings.

To assess the imidazole propionate-producing potential of pure bacterial cultures, all strains were maintained on BHI medium (as described above) without histidine or urocanate under strict anaerobic conditions in a Coy chamber (5% hydrogen, 10% carbon dioxide and 85% nitrogen) at 37°C. To test their potential to produce imidazole propionate, a single colony of each bacterial strain was inoculated in BHI medium and, after overnight incubation, 1% of the preculture was inoculated in BHI medium supplemented with 10 mM histidine or urocanate and incubated for 24 h. After incubation, the cell-free supernatant was used for quantification of imidazole propionate.

### Quantitative real-time PCR analysis

Mouse liver was homogenized in RLT buffer with β-mercaptoethanol using TissueLyser (Qiagen, Hilden, Germany). RNA was isolated using the RNeasy Minikit (Qiagen) and reverse transcribed to synthesize cDNA using High Capacity cDNA Reverse Transcription Kit (Applied Biosystems, Foster City, CA, USA) according to the manufacturer's protocol. Real-time PCR was performed using 1X SYBR Green Master Mix (Thermo Scientific, Waltham, MA, USA) using the following primers.
L32: 5'-CCTCTGGTGAAGCCCAAGATC-3' (SEQ ID NO: 1) and 5'-TCTGGGTTTCCGCCAGTTT-3' (SEQ ID NO: 2);
Pepck: 5'-GGCCACAGCTGCTGCAG-3' (SEQ ID NO: 3) and 5'-GGTCGCATGGCAAAGGG-3' (SEQ ID NO: 4);
G6pase: 5'-CTGTGAGACCGGACCAGGA-3' (SEQ ID NO: 5) and 5'-GACCATAACATAGTATACACCTGCTGC-3' (SEQ ID NO: 6);
Fasn: 5'-CGGAAACTTCAGGAAATGTCC-3' (SEQ ID NO: 7) and 5'-TCAGAGACGTGTCACTCCTGG-3' (SEQ ID NO: 8);
Srebp1c: 5'-AGCCATGGATTGCACATTTGA-3' (SEQ ID NO: 9) and 5'-CAAATAGGCCAGGGAAGTCA-3' (SEQ ID NO: 10);
Irs1: 5'-GTGAACCTCAGTCCCAACCATAAC-3' (SEQ ID NO: 11) and 5'- CCGGCACCCTTGAGTGTCT-3' (SEQ ID NO: 12);
Irs2: 5'-TCCCACATCGGGCTTGAA-3' (SEQ ID NO: 13) and 5'-CTGCACGGATGACCTTAGCA-3' (SEQ ID NO: 14).

### Preparation of protein extracts

Snap-frozen liver and harvested cells were lysed in buffer containing 50 mM Tris-HCI (pH 7.4), 150 mM NaCl, 1 mM EDTA, 1 mM Na₃VO₄, 20 mM NaF, 10 mM glycerophosphate, 1 mM PMSF, 10 % glycerol, 1 % Triton X-100 and protease inhibitor cocktail. For immunoprecipitation, the cell lysates were obtained in 0.3 % CHAPS-containing lysis buffer, sonicated and centrifuged at 20,000 g for 15 min at 4°C, and the supernatant was incubated with 20 µl of anti-FLAG beads (Sigma) at 4°C for 4 h under gentle agitation.

### Primary hepatocytes

Primary hepatocytes were isolated from C57BL/6J mice by digesting liver with perfusion of collagenase type IV as described previously [2]. After perfusion, 16 × 10⁵ cells were plated on collagen-coated 60 mm dishes in Dulbecco's modified Eagle's medium (DMEM)/F12 supplemented with fetal bovine serum (FBS), penicillin/streptomycin and 100 nM dexamethasone. After 4 h, the medium was changed to DMEM/F12 containing penicillin/streptomycin.

For insulin stimulation, primary hepatocytes were cultured in DMEM/F12 with or without 100 µM imidazole propionate for 14 to 24 h and treated with 5 nM insulin for the indicated times. For amino acid deprivation, primary hepatocytes were cultured in DMEM/F12 for 24 h, rinsed once with Earle's Balanced Salt Solution (EBSS) and then incubated in EBSS for 1 h before incubation with 100 µM imidazole propionate for the indicated times.

### HEK293 cells

HEK293 cells (ATCC CRL-1573) were grown and maintained in high-glucose (4.5 g/l glucose) DMEM with 10 % (v/v) FBS. All HEK293 cell experiments were performed in serum-starved and amino acid-deprived conditions. 24 h after transfection (with plasmids or siRNA as indicated), HEK293 cells cultured in poly-L-lysine-coated 60 mm dishes were rinsed twice with DMEM without FBS and incubated in serum-free DMEM for 18 h. HEK293 cells were then washed once with EBSS and incubated in EBSS for 1 h. For insulin stimulation, serum- and amino acid-starved HEK293 cells were preincubated with imidazole propionate in EBSS for 2 or 8 h and treated with 5 nM insulin in EBSS.

### Antibodies and reagents

Antibodies to phospho-Akt (S473, T308), phospho-S6K1 (T389), phospho-IRS (S636/S639), phospho-p62 (T269/S272), phospho-mTOR (S2481), mTOR, DEPTOR, p62, IR, IRS-2, p38y, Akt, GAPDH and Actin were purchased from Cell Signaling (Danvers, MA). Anti-IRS-1 was obtained from Millipore (Darmstadt, Germany). Antibodies to p38δ, Myc and HA were acquired from Santa Cruz Biotechnology (Santa Cruz, CA, USA). Anti-FLAG was purchased from Sigma-Aldrich (St. Louis, MO, USA). Anti-LAMP2 was obtained from Abcam (ab25631). Imidazole propionate (deamino histidine, CAS 1074-59-5, sc294276), urocanate (4-imidazoleacrylic acid, CAS 104-89-3, sc214246), histidine (CAS 71-00-1, sc394101) and indole propionate (CAS 830-96-6, sc255215) were purchased from Santa Cruz Biotechnology. Glutamate, insulin and SP600125 were from Sigma-Aldrich. Rapamycin and BIRB796 were obtained from Merck Millipore and BI78D3 was acquired from Tocris.

### Plasmids and siRNAs

FLAG mTOR and HA Raptor were kindly provided by Dr Sung Ho Ryu (Pohang University of Science and Technology, Pohang, South Korea). Myc-Rag GTPases constructs were gifts from Dr Do-Hyung Kim (University of Minnesota, MN, USA). AllStars negative control siRNA, p38δ siRNA1 (Hs_MAPK13_5 targeting CCGGAGTGGCATGAAGCTGTA (SEQ ID NO: 15)), p38δ siRNA2 (Hs_MAPK13_6 targeting CGGGATGAGCCTCATCCGGAA (SEQ ID NO: 16)), p38y siRNA1 (Hs_MAPK12_5 targeting CTGGACGTATTCACTCCTGAT (SEQ ID NO: 17)), p38y siRNA2 (Hs_MAPK12_8 targeting CTGGGAGGTGCGCGCCGTGTA (SEQ ID NO: 18)), p38α siRNA (Hs_MAPK14_5 targeting AACTGCGGTTACTTAAACATA (SEQ ID NO: 21)), p38β siRNA (Hs_MAPK11_6 targeting CTGAGCGACGAGCACGTTCAA (SEQ ID NO: 22)), S6K1 siRNA (Hs_RPS6KB1_5 targeting GGGAGTTGGACCATATGAACT (SEQ ID NO: 23)) and Raptor siRNA (Hs_RPTOR_1 targeting GACACGGAAGATGTTCGACAA (SEQ ID NO: 24)) (Qiagen, Valencia, CA) were transfected into HEK293 cells using Lipofectamin 2000 according to the manufacturer's instructions.

### Immunofluorescence

Cells were grown on poly-L-lysine (Sigma) coated 4 well chamber slides, fixed with 4 % paraformaldehyde for 15 min at 37°C, then washed with glycine-PBS and permeablized with 0.3 % Triton X-100 for 10 min. After blocking with 3 % BSA in PBS, cells were stained for mTOR and LAMP2. Images were obtained by confocal microscope.

### Preparation of protein extracts for detecting ubiquitination

Cells were lysed under denaturing condition with 100 µl buffer A containing 2% SDS, and the cell lysates were transferred to a 1.5-ml microcentrifuge tube. The tube was placed on a hot plate immediately at 95°C for 10 min. The cell lysates were sonicated and 700 µl of buffer A without SDS was added. The samples were incubated at 4°C for 30 min, followed by centrifugation (20,000 g, 20 min). The supernatant was incubated with 20 µl of anti-FLAG beads (Sigma) at 4°C for 4 h under gentle agitation.

### mTORC2 kinase activity assay

HEK293 cells were transfected with 1 µg of HA Rictor and 0.5 µg of Flag mTOR for mTORC2 activity assay, lysed with 0.3% CHAPS-containing lysis buffer, sonicated and centrifuged at 20,000 g for 15 min at 4°C, and the supernatant was incubated with 2 µg of anti-HA antibody at 4°C for overnight under gentle agitation. Immunocomplexes were then collected with protein A-Sepharose beads (RepliGen, Needham, MA) at 4°C for 2 h and washed three times with 0.3% CHAPS-containing lysis buffer. Immunoprecipitates were preincubated with 450 ng of inactive Akt1 (abcam, ab116412) in kinase assay buffer [12.5 mM MOPS (pH 7.2), 10 mM beta-glycerol-phosphate, 12.5 mM MgCl₂, 2.5 mM EGTA, and 1 mM EDTA] for 10 min on ice. Kinase reaction was started by adding 100 µM ATP for 15 min at 37°C and stopped by adding 5x Laemmli buffer.

### In vitro kinase assay

400 ng of recombinant p62 (Abcam, ab95320) and 200 ng of recombinant p38y (Abcam, ab125651) were incubated in a kinase assay buffer [25 mM MOPS (pH 7.2), 12.5 mM beta-glycerol-phosphate, 25 mM MgCl₂, 5 mM EGTA, 2 mM EDTA and 1 mM DTT] for 15 min on ice. In a separate tube, 1 mM ATP diluted in a kinase assay buffer was incubated with DMSO or imidazole propionate (final concentration of ATP in the kinase reaction was 50 or 200 µM). The kinase reaction was started by adding ATP with DMSO or imidazole propionate to the p38y and p62 mixture for 10 min at 30°C, and stopped by adding 5× Laemmli buffer.

### Statistical analysis

Wilcoxon rank-sum test was used to test if metabolites in portal blood differed in abundance between subjects with and without type 2 diabetes (untargeted metabolomics). The raw P values were adjusted by the Benjamini-Hochberg method [3] with a FDR of 0.1. Kruskal-Wallis rank-sum test followed by Dunnett's multiple comparisons was used for targeted comparisons of imidazole propionate among groups with different levels of glucose tolerance from a large Swedish cohort. Multiple regression and analysis of variance with corrections for confounding factors BMI, age and sex were used for this larger Swedish cohort. Analysis of datasets containing multiple measurements from mice and humans (comparing metabolites in portal and cava blood plasma) was performed with a two-way ANOVA with repeated measurements. Wilcoxon one-tailed test was done to see if type 2 diabetes-associated microbiota produces higher imidazole propionate from histidine. One-way ANOVA with Tukey's test was performed when comparing three or more groups. Analysis of effects of imidazole propionate on concentrations of different metabolites or mice physiology (energy expenditure or locomotor activity) depending on light/dark cycle was done with a two-way ANOVA with Sidak's multiple comparisons test. Otherwise, unpaired two-tailed Student's t tests were used, as indicated.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

### REFERENCES

[1] Anjani, K. et al. Circulating phospholipid profiling identifies portal contribution to NASH signature in obesity. J Hepatol 62, 905-912 (2015).
[2] Zhang, W. S. et al. PCB 126 and Other Dioxin-Like PCBs Specifically Suppress Hepatic PEPCK Expression via the Aryl Hydrocarbon Receptor. PloS one 7, doi:ARTN e3710310.1371/journal.pone.0037103 (2012).
[3] Benjamini, Y. & Hochberg, Y. Controlling the False Discovery Rate - a Practical and Powerful Approach to Multiple Testing. J Roy Stat Soc B Met 57, 289-300 (1995).
[4] Hug, D.H. et al. Regulation of Histidine Catabolism by Succinate in Pseudomonas putida. J Bacteriol, 96(2), 396-402 (1968)

## Claims

1. A method of determining whether a subject is suffering from, or having a risk of suffering from, type 2 diabetes (T2D) or impaired glucose tolerance (IGT), said method comprising:
determining an amount of imidazole propionate in a body sample from said subject;
determining an amount of urocanate in said body sample; and
determining whether said subject is suffering from, or having a risk of suffering from, type 2 diabetes or impaired glucose tolerance based on said determined amount of imidazole propionate and said determined amount of urocanate.

2. The method according to claim 1, wherein determining said amount of imidazole propionate comprises determining said amount of imidazole propionate in a body fluid sample from said subject, preferably a body fluid sample selected from a group consisting of a blood sample, a plasma sample, a serum sample, a urine sample and a fecal sample from said subject, and more preferably a body fluid sample selected from a group consisting of a blood sample, a plasma sample and a serum sample from said subject.

3. The method according to claim 1 or 2, wherein
determining an amount of imidazole propionate comprises determining a concentration of imidazole propionate in said body sample; and
determining whether said subject is suffering from, or having a risk of suffering from, type 2 diabetes or impaired glucose tolerance comprises:
comparing said concentration of imidazole propionate with a threshold concentration within an interval of 10.7 nM and 13.8 nM; and
determining that said subject is suffering from, or having a risk of suffering from, type 2 diabetes if said concentration of imidazole propionate is equal to or larger than said threshold concentration.

4. The method according to any of the claims 1 to 3, wherein determining whether said subject is suffering from, or having a risk of suffering from, type 2 diabetes or impaired glucose tolerance comprises determining whether said subject is suffering from, or having a risk of suffering from, type 2 diabetes or impaired glucose tolerance based on a ratio between said determined amount of imidazole propionate and said determined amount of urocanate.

5. The method according to claim 4, wherein determining whether said subject is suffering from, or having a risk of suffering from, type 2 diabetes or impaired glucose tolerance comprises:
comparing said ratio with a threshold ratio within an interval of 0.15 and 0.325, preferably within an interval of 0.17 and 0.3, and more preferably within an interval of 0.2 and 0.3; and
determining that said subject is suffering from, or having a risk of suffering from, type 2 diabetes if said ratio is equal to or larger than said threshold ratio.

6. A method of determining whether a subject is suffering from, or having a risk of suffering from, type 2 diabetes (T2D) or impaired glucose tolerance (IGT), said method comprising:
determining an amount of imidazole propionate producing bacteria in a body sample from said subject; and
determining whether said subject is suffering from, or having a risk of suffering from, type 2 diabetes or impaired glucose tolerance based on said determined amount of imidazole propionate producing bacteria.

7. A method of identifying an agent effective in preventing, inhibiting or treating type 2 diabetes or impaired glucose tolerance in a subject, said method comprising:
adding an agent to an *in vitro* simulated human intestinal model challenged with histidine and/or urocanate;
measuring an amount of imidazole propionate produced by said *in vitro* simulated human intestinal model; and
identifying said agent as effective in preventing, inhibiting or treating type 2 diabetes or impaired glucose tolerance based on said amount of imidazole propionate.

8. The method according to claim 7, wherein adding said agent comprises adding said agent to said *in vitro* simulated human intestinal model inoculated with an aliquot of feces from a subject suffering from type 2 diabetes or impaired glucose tolerance.

9. The method according to claim 7 or 8, wherein identifying said agent as effective in preventing, inhibiting or treating type 2 diabetes or impaired glucose tolerance comprises:
comparing said amount of imidazole propionate with a reference amount of imidazole propionate produced by an *in vitro* reference simulated human intestinal model challenged with histidine and/or urocanate following addition of said agent, said *in vitro* reference simulated human intestinal model lacks any urocanate reductase producing bacteria or is inoculated with an aliquot of feces from a subject with normal glucose tolerance; and
identifying said agent as effective in preventing, inhibiting or treating type 2 diabetes or impaired glucose tolerance based a comparison of said amount of imidazole propionate and said reference amount of imidazole propionate.

10. The method according to claim 7 or 8, wherein identifying said agent as effective in preventing, inhibiting or treating type 2 diabetes or impaired glucose tolerance comprises:
comparing said amount of imidazole propionate with a reference amount of imidazole propionate produced by said *in vitro* simulated human intestinal model challenged with histidine and/or urocanate but in in absence said agent; and
identifying said agent as effective in preventing, inhibiting or treating type 2 diabetes or impaired glucose tolerance based a comparison of said amount of imidazole propionate and said reference amount of imidazole propionate.

11. The method according to any of the claims 7 to 10, further comprising measuring an amount of urocanate accumulated in said *in vitro* simulated human intestinal model, wherein identifying said agent as effective in preventing, inhibiting or treating type 2 diabetes or impaired glucose tolerance comprises identifying said agent as effective in preventing, inhibiting or treating type 2 diabetes or impaired glucose tolerance based on said amount of imidazole propionate and said amount of urocanate, preferably based on a ratio said amount of imidazole propionate and said amount of urocanate.

## Patentansprüche

1. Verfahren zum Bestimmen, ob ein Proband an Typ-2-Diabetes (T2D) oder gestörter Glukosetoleranz (IGT) leidet oder ein Risiko aufweist, daran zu leiden, wobei das Verfahren umfasst:
Bestimmen einer Menge von Imidazolpropionat in einer Körperprobe von dem Probanden;
Bestimmen einer Menge von Urocanat in der Körperprobe und
Bestimmen, ob der Proband an Typ-2-Diabetes oder gestörter Glukosetoleranz leidet oder ein Risiko aufweist, daran zu leiden, auf der Basis der bestimmten Menge von Imidazolpropionat und der bestimmten Menge von Urocanat.

2. Verfahren nach Anspruch 1, wobei das Bestimmen der Menge von Imidazolpropionat ein Bestimmen der Menge von Imidazolpropionat in einer Körperflüssigkeitsprobe von dem Probanden umfasst, vorzugsweise einer Körperflüssigkeitsprobe, die aus einer Gruppe bestehend aus einer Blutprobe, einer Plasmaprobe, einer Serumprobe, einer Urinprobe und einer Stuhlprobe von dem Probanden ausgewählt ist, und mehr bevorzugt einer Körperflüssigkeitsprobe, die aus einer Gruppe bestehend aus einer Blutprobe, einer Plasmaprobe und einer Serumprobe von dem Probanden ausgewählt ist.

3. Verfahren nach Anspruch 1 oder 2, wobei
das Bestimmen einer Menge von Imidazolpropionat ein Bestimmen einer Konzentration von Imidazolpropionat in der Körperprobe umfasst und
das Bestimmen, ob der Proband an Typ-2-Diabetes oder gestörter Glukosetoleranz leidet oder ein Risiko aufweist, daran zu leiden, umfasst:
Vergleichen der Konzentration von Imidazolpropionat mit einer Grenzkonzentration innerhalb eines Intervalls von 10,7 nM und 13,8 nM und
Bestimmen, ob der Proband an Typ-2-Diabetes leidet oder ein Risiko aufweist, daran zu leiden, wenn die Konzentration von Imidazolpropionat gleich oder größer als die Grenzkonzentration ist.

4. Verfahren nach einem der Ansprüche 1 bis 3, wobei das Bestimmen, ob der Proband an Typ-2-Diabetes oder gestörter Glukosetoleranz leidet oder ein Risiko aufweist, daran zu leiden, ein Bestimmen, ob der Proband an Typ-2-Diabetes oder gestörter Glukosetoleranz leidet oder ein Risiko aufweist, daran zu leiden, auf der Basis eines Verhältnisses zwischen der bestimmten Menge von Imidazolpropionat und der bestimmten Menge von Urocanat umfasst.

5. Verfahren nach Anspruch 4, wobei das Bestimmen, ob der Proband an Typ-2-Diabetes oder gestörter Glukosetoleranz leidet oder ein Risiko aufweist, daran zu leiden, umfasst:
Vergleichen des Verhältnisses mit einem Grenzverhältnis innerhalb eines Intervalls von 0,15 und 0,325, vorzugsweise innerhalb eines Intervalls von 0,17 und 0,3 und mehr bevorzugt innerhalb eines Intervalls von 0,2 und 0,3; und
Bestimmen, ob der Proband an Typ-2-Diabetes leidet oder ein Risiko aufweist, daran zu leiden, wenn das Verhältnis gleich oder größer als das Grenzverhältnis ist.

6. Verfahren zum Bestimmen, ob ein Proband an Typ-2-Diabetes (T2D) oder gestörter Glukosetoleranz (IGT) leidet oder ein Risiko aufweist, daran zu leiden, wobei das Verfahren umfasst:
Bestimmen einer Menge von Imidazolpropionat produzierenden Bakterien in einer Körperprobe von dem Probanden und
Bestimmen, ob der Proband an Typ-2-Diabetes oder gestörter Glukosetoleranz leidet oder ein Risiko aufweist, daran zu leiden, auf der Basis der bestimmten Menge von Imidazolpropionat produzierenden Bakterien.

7. Verfahren zum Identifizieren eines Agens, das beim Verhindern, Hemmen oder Behandeln von Typ-2-Diabetes oder gestörter Glukosetoleranz bei einem Probanden wirksam ist, wobei das Verfahren umfasst:
Zugeben eines Agens zu einem simulierten *In-vitro-*Modell des menschlichen Darms, das mit Histidin und/oder Urocanat belastet wird;
Messen einer Menge von Imidazolpropionat, die von dem simulierten *In-vitro-Modell* des menschlichen Darms produziert wird; und
Identifizieren des Agens als beim Verhindern, Hemmen oder Behandeln von Typ-2-Diabetes oder gestörter Glukosetoleranz bei einem Probanden wirksam auf der Basis der Menge von Imidazolpropionat.

8. Verfahren nach Anspruch 7, wobei das Zugeben des Agens ein Zugeben des Agens zu dem simulierten *In-vitro-Modell* des menschlichen Darms mit einem Stuhl-Aliquot von einem Probanden, der an Typ-2-Diabetes oder gestörter Glukosetoleranz leidet, inokuliert umfasst.

9. Verfahren nach Anspruch 7 oder 8, wobei das Identifizieren des Agens als beim Verhindern, Hemmen oder Behandeln von Typ-2-Diabetes oder gestörter Glukosetoleranz wirksam umfasst:
Vergleichen der Menge von Imidazolpropionat mit einer Referenzmenge von Imidazolpropionat, die von dem simulierten *In-vitro*-Referenzmodell des menschlichen Darms produziert wird, das mit Histidin und/oder Urocanat belastet wird, nach der Zugabe des Agens, wobei es dem simulierten *In-vitro*-Referenzmodell des menschlichen Darms an jeglichen Urocanat-Reduktase produzierenden Bakterien fehlt oder es mit einem Stuhl-Aliquot von einem Probanden mit normaler Glukosetoleranz inokuliert wird; und
Identifizieren des Agens als beim Verhindern, Hemmen oder Behandeln von Typ-2-Diabetes oder gestörter Glukosetoleranz bei einem Probanden wirksam auf der Basis eines Vergleichs der Menge von Imidazolpropionat und der Referenzmenge von Imidazolpropionat.

10. Verfahren nach Anspruch 7 oder 8, wobei das Identifizieren des Agens als beim Verhindern, Hemmen oder Behandeln von Typ-2-Diabetes oder gestörter Glukosetoleranz wirksam umfasst:
Vergleichen der Menge von Imidazolpropionat mit einer Referenzmenge von Imidazolpropionat, die von dem simulierten *In-vitro*-Modell des menschlichen Darms produziert wird, das mit Histidin und/oder Urocanat belastet wird, jedoch in Abwesenheit des Agens; und
Identifizieren des Agens als beim Verhindern, Hemmen oder Behandeln von Typ-2-Diabetes oder gestörter Glukosetoleranz bei einem Probanden wirksam auf der Basis eines Vergleichs der Menge von Imidazolpropionat und der Referenzmenge von Imidazolpropionat.

11. Verfahren nach einem der Ansprüche 7 bis 10, weiterhin umfassend ein Messen einer Menge von Urocanat, die sich in dem simulierten *In-vitro-*Modell des menschlichen Darms angesammelt hat, wobei das Identifizieren des Agens als beim Verhindern, Hemmen oder Behandeln von Typ-2-Diabetes oder gestörter Glukosetoleranz bei einem Probanden wirksam ein Identifizieren des Agens als beim Verhindern, Hemmen oder Behandeln von Typ-2-Diabetes oder gestörter Glukosetoleranz bei einem Probanden wirksam auf der Basis der Menge von Imidazolpropionat und der Menge von Urocanat, vorzugsweise auf der Basis eines Verhältnisses der Menge von Imidazolpropionat und der Menge von Urocanat umfasst.

## Revendications

1. Procédé pour déterminer si un sujet souffre, ou présente un risque de souffrir, d'un diabète de type 2 (T2D) ou d'une tolérance au glucose altérée (IGT), ledit procédé comprenant :
déterminer une quantité de propionate d'imidazole dans un échantillon corporel provenant dudit sujet ;
déterminer une quantité d'urocanate dans ledit échantillon corporel ; et
déterminer si ledit sujet souffre, ou présente un risque de souffrir, d'un diabète de type 2 ou d'une tolérance au glucose altérée sur la base de ladite quantité déterminée de propionate d'imidazole et de ladite quantité déterminée d'urocanate.

2. Procédé selon la revendication 1, dans lequel la détermination de ladite quantité de propionate d'imidazole comprend déterminer ladite quantité de propionate d'imidazole dans un échantillon de fluide corporel provenant dudit sujet, de préférence un échantillon de fluide corporel choisi dans un groupe constistant en un échantillon sanguin, un échantillon de plasma, un échantillon de sérum, un échantillon d'urine et un échantillon de matières fécales provenant dudit sujet, et de façon davantage préférée un échantillon de fluide corporel choisi dans un groupe consistant en un échantillon sanguin, un échantillon de plasma et un échantillon de sérum provenant dudit sujet.

3. Procédé selon l'une des revendications 1 ou 2, dans lequel :
déterminer une quantité de propionate d'imidazole comprend déterminer une concentration de propionate d'imidazole dans ledit échantillon corporel ; et
déterminer si ledit sujet souffre, ou présente un risque de souffrir, d'un diabète de type 2 ou d'une tolérance au glucose altérée comprend :
comparer ladite concentration de propionate d'imidazole avec une concentration seuil dans un intervalle de 10,7 nM et 13,8 nM ; et
déterminer que ledit sujet souffre, ou présente un risque de souffrir, d'un diabète de type 2 si ladite concentration de propionate d'imidazole est égale ou supérieure à ladite concentration seuil.

4. Procédé selon l'une quelconque des revendications 1 à 3, dans laquel déterminer si ledit sujet souffre, ou présente un risque de souffrir, d'un diabète de type 2 ou d'une tolérance au glucose altérée comprend déterminer si ledit sujet souffre, ou présente un risque de souffrir, d'un diabète de type 2 ou d'une tolérance au glucose altérée sur la base d'un rapport entre ladite quantité déterminée de propionate d'imidazole et ladite quantité déterminée d'urocanate.

5. Procédé selon la revendication 4, dans lequel déterminer si ledit sujet souffre, ou présente un risque de souffrir, d'un diabète de type 2 ou d'une tolérance au glucose altérée comprend :
comparer ledit rapport à un rapport seuil dans un intervalle de 0,15 et 0,325, de préférence dans un intervalle de 0,17 et 0,3, et de façon davantage préférée dans un intervalle de 0,2 et 0,3 ; et
déterminer que ledit sujet souffre, ou présente un risque de souffrir, d'un diabète de type 2 si ledit rapport est égal ou supérieur audit rapport seuil.

6. Procédé pour déterminer si un sujet souffre, ou présente un risque de souffrir, d'un diabète de type 2 (T2D) ou d'une tolérance au glucose altérée (IGT), ledit procédé comprenant :
déterminer une quantité de bactéries produisant du propionate d'imidazole dans un échantillon corporel provenant dudit sujet ; et
déterminer si ledit sujet souffre, ou présente un risque de souffrir, d'un diabète de type 2 ou d'une tolérance au glucose altérée sur la base de ladite quantité déterminée de bactéries produisant du propionate d'imidazole.

7. Procédé d'identification d'un agent efficace pour prévenir, inhiber ou traiter un diabète de type 2 ou une tolérance au glucose altérée chez un sujet, ledit procédé comprenant :
ajouter un agent à un modèle intestinal humain simulé *in vitro* stimulé par de l'histidine et/ou de l'urocanate ;
mesurer une quantité de propionate d'imidazole produit par ledit modèle intestinal humain simulé *in vitro* ; et
identifier ledit agent comme étant efficace pour prévenir, inhiber ou traiter un diabète de type 2 ou une tolérance au glucose altérée sur la base de ladite quantité de propionate d'imidazole.

8. Procédé selon la revendication 7, dans lequel l'ajout dudit agent comprend ajouter ledit agent audit modèle intestinal humain simulé *in vitro* inoculé avec une quantité aliquote de matières fécales provenant d'un sujet souffrant d'un diabète de type 2 ou d'une tolérance au glucose altérée.

9. Procédé selon l'une des revendication 7 ou 8, dans lequel l'identification dudit agent comme étant efficace pour prévenir, inhiber ou traiter un diabète de type 2 ou une tolérance au glucose altérée comprend :
comparer ladite quantité de propionate d'imidazole avec une quantité de référence de propionate d'imidazole produit par un modèle intestinal humain simulé *in vitro* de référence stimulé avec de l'histidine et/ou de l'urocanate suivant l'ajout dudit agent, ledit modèle intestinal humain simulé *in vitro* de référence étant dépourvu de toute bactérie produisant de l'urocanate réductase ou étant inoculé avec une quantité aliquote de matières fécales provenant d'un sujet ayant une tolérance normale au glucose ; et
identifier ledit agent comme étant efficace pour prévenir, inhiber ou traiter un diabète de type 2 ou une tolérance au glucose altérée sur la base d'une comparaison de ladite quantité de propionate d'imidazole et de ladite quantité de référence de propionate d'imidazole.

10. Procédé selon l'une des revendications 7 ou 8, dans lequel l'identification dudit agent comme étant efficace pour prévenir, inhiber ou traiter un diabète de type 2 ou une tolérance au glucose altérée comprend :
comparer ladite quantité de propionate d'imidazole avec une quantité de référence de propionate d'imidazole produit par ledit modèle intestinal humain simulé *in vitro* stimulé avec de l'histidine et/ou de l'urocanate mais en l'absence dudit agent ; et
identifier ledit agent comme étant efficace pour prévenir, inhiber ou traiter un diabète de type 2 ou une tolérance au glucose altérée sur la base d'une comparaison de ladite quantité de propionate d'imidazole et de ladite quantité de référence de propionate d'imidazole.

11. Procédé selon l'une quelconque des revendications 7 à 10, comprenant en outre la mesure d'une quantité d'urocanate accumulée dans ledit modèle intestinal humain simulé *in vitro,* dans lequel l'identification dudit agent comme étant efficace pour prévenir, inhiber ou traiter un diabète de type 2 ou une tolérance au glucose altérée comprend identifier ledit agent comme étant efficace pour prévenir, inhiber ou traiter un diabète de type 2 ou une tolérance au glucose altérée sur la base de ladite quantité de propionate d'imidazole et de ladite quantité d'urocanate, de préférence sur la base d'un rapport entre ladite quantité de propionate d'imidazole et ladite quantité d'urocanate.


### SEQUENCE LISTING

<110> KOH, Ara
   BÄCKHED, Fredrik
<120> METABOLIC DISORDERS
<130> P1369PC00
<150> SE 1651551-2
   <151> 2016-11-25
<160> 24
<170> PatentIn version 3.5
<210> 1
   <211> 21
   <212> DNA
   <213> Artificial Sequence
<220>
   <223> PCR primer
<400> 1
   cctctggtga agcccaagat c 21
<210> 2
   <211> 19
   <212> DNA
   <213> Artificial Sequence
<220>
   <223> PCR primer
<400> 2
   tctgggtttc cgccagttt 19
<210> 3
   <211> 17
   <212> DNA
   <213> Artificial Sequence
<220>
   <223> PCR primer
<400> 3
   ggccacagct gctgcag 17
<210> 4
   <211> 17
   <212> DNA
   <213> Artificial Sequence
<220>
   <223> PCR primer
<400> 4
   ggtcgcatgg caaaggg 17
<210> 5
   <211> 19
   <212> DNA
   <213> Artificial Sequence
<220>
   <223> PCR primer
<400> 5
   ctgtgagacc ggaccagga 19
<210> 6
   <211> 27
   <212> DNA
   <213> Artificial Sequence
<220>
   <223> PCR primer
<400> 6
   gaccataaca tagtatacac ctgctgc 27
<210> 7
   <211> 21
   <212> DNA
   <213> Artificial Sequence
<220>
   <223> PCR primer
<400> 7
   cggaaacttc aggaaatgtc c 21
<210> 8
   <211> 21
   <212> DNA
   <213> Artificial Sequence
<220>
   <223> PCR primer
<400> 8
   tcagagacgt gtcactcctg g 21
<210> 9
   <211> 21
   <212> DNA
   <213> Artificial Sequence
<220>
   <223> PCR primer
<400> 9
   agccatggat tgcacatttg a 21
<210> 10
   <211> 20
   <212> DNA
   <213> Artificial Sequence
<220>
   <223> PCR primer
<400> 10
   caaataggcc agggaagtca 20
<210> 11
   <211> 24
   <212> DNA
   <213> Artificial Sequence
<220>
   <223> PCR primer
<400> 11
   gtgaacctca gtcccaacca taac 24
<210> 12
   <211> 19
   <212> DNA
   <213> Artificial Sequence
<220>
   <223> PCR primer
<400> 12
   ccggcaccct tgagtgtct 19
<210> 13
   <211> 18
   <212> DNA
   <213> Artificial Sequence
<220>
   <223> PCR primer
<400> 13
   tcccacatcg ggcttgaa 18
<210> 14
   <211> 20
   <212> DNA
   <213> Artificial Sequence
<220>
   <223> PCR primer
<400> 14
   ctgcacggat gaccttagca 20
<210> 15
   <211> 21
   <212> DNA
   <213> Homo sapiens
<400> 15
   ccggagtggc atgaagctgt a 21
<210> 16
   <211> 21
   <212> DNA
   <213> Homo sapiens
<400> 16
   cgggatgagc ctcatccgga a 21
<210> 17
   <211> 21
   <212> DNA
   <213> Homo sapiens
<400> 17
   ctggacgtat tcactcctga t 21
<210> 18
   <211> 21
   <212> DNA
   <213> Homo sapiens
<400> 18
   ctgggaggtg cgcgccgtgt a 21
<210> 19
   <211> 17
   <212> DNA
   <213> Artificial Sequence
<220>
   <223> PCR primer
<400> 19
   gtttgatcct ggctcag 17
<210> 20
   <211> 18
   <212> DNA
   <213> Artificial Sequence
<220>
   <223> PCR primer
<400> 20
   cggctacctt gttacgac 18
<210> 21
   <211> 21
   <212> DNA
   <213> Homo sapiens
<400> 21
   aactgcggtt acttaaacat a 21
<210> 22
   <211> 21
   <212> DNA
   <213> Homo sapiens
<400> 22
   ctgagcgacg agcacgttca a 21
<210> 23
   <211> 21
   <212> DNA
   <213> Homo sapiens
<400> 23
   gggagttgga ccatatgaac t 21
<210> 24
   <211> 21
   <212> DNA
   <213> Homo sapiens
<400> 24
   gacacggaag atgttcgaca a 21